# NEUE EUROPÄISCHE PATENTSCHRIFT

(11) **EP 2 396 655 B2**
(45) Veröffentlichungstag und Bekanntmachung des Hinweises auf die Entscheidung über den Einspruch: **06.12.2017**
(45) Hinweis auf die Patenterteilung: 04.03.2015
(21) Anmeldenummer: 10705125.2
(22) Anmeldetag: 11.02.2010
(51) Int. Cl.: G01N 33/53, G01N 33/569

(54) **VORRICHTUNG ZUM SEROLOGISCHEN NACHWEIS VON YERSINIEN-INFEKTIONEN UND/ODER DEREN FOLGEERKRANKUNGEN SOWIE VERWENDUNG DER PROTEINE MyfA UND PsaA VON Y.ENTEROCOLITICA UND Y.PSEUDOTUBERCULOSIS ALS REKOMBINANTE ANTIGENE**
DEVICE FOR SEROLOGICAL INVESTIGATION OF YERSINIA INFECTIONS AND/OR SUBSEQUENT ILLNESSES AND USE OF PROTEINS MYFA AND PSAA OF Y ENTEROCOLITICA AND Y. PSEUDOTUBERCULOSIS AS RECOMBINANT ANTIGENS
DISPOSITIF DE VÉRIFICATION SÉROLOGIQUE D'INFECTIONS DE YERSINIA ET/OU LEURS MALADIES SUBSÉQUENTES ET UTILISATION DES PROTÉINES MYFA ET PSAA DE Y. ENTEROCOLITICA ET Y. PSEUDOTUBERCULOSIS EN TANT QU'ANTIGÈNES RECOMBINÉS

(30) Priorität: 16.02.2009 EP 09002105
(43) Veröffentlichungstag der Anmeldung: 21.12.2011
(73) Patentinhaber: Mikrogen GmbH, 82061 Neuried (DE)
(72) Erfinder: SOUTSCHEK, Erwin, 83225 Berg (DE)
(74) Vertreter: Keller, Günter
(86) Internationale Anmeldenummer: PCT/EP2010/051673
(87) Internationale Veröffentlichungsnummer: WO 2010/092093

(56) Entgegenhaltungen:
- IRIARTE M ET AL: "Molecular determinants of Yersinia pathogenesis" MICROBIOLOGIA, MADRID, ES, Bd. 12, Nr. 2, 1. Juni 1996 (1996-06-01), Seiten 267-270, XP009116676 ISSN: 0213-4101
- TOMASO H ET AL: "Seroprevalence of Anti-Yersinia Antibodies in Healthy Austrians" EUROPEAN JOURNAL OF EPIDEMIOLOGY, KLUWER ACADEMIC PUBLISHERS, DO, Bd. 21, Nr. 1, 1. Januar 2006 (2006-01-01) , Seiten 77-81, XP019239148 ISSN: 1573-7284
- STOLK-ENGELAAR V M ET AL: "Clinical presentation and diagnosis of gastrointestinal infections by Yersinia enterocolitica in 261 Dutch patients." SCANDINAVIAN JOURNAL OF INFECTIOUS DISEASES 1996, Bd. 28, Nr. 6, 1996, Seiten 571-575, XP002529751 ISSN: 0036-5548
- HEESEMANN J: "[Enteropathogenic yersinias: pathogenicity factors and new diagnostic methods]" IMMUNITÄT UND INFEKTION DEC 1990, Bd. 18, Nr. 6, Dezember 1990 (1990-12), Seiten 186-191, XP009117342 ISSN: 0340-1162
- LEIVA J ET AL: "Coagglutination with antisera to MyfA and PsaA to distinguish Yersinia enterocolitica from Yersinia pseudotuberculosis pathogenic isolates" CONTRIBUTIONS TO MICROBIOLOGY AND IMMUNOLOGY; YERSINIOSIS: PRESENT AND FUTURE S. KARGER AG, P.O. BOX, ALLSCHWILERSTRASSE 10, CH-4009 BASEL, SWITZERLAND; S. KARGER AG, NEW YORK, NEW YORK, USA SERIES : CONTRIBUTIONS TO MICROBIOLOGY AND IMMUNOLOGY (ISSN, 1995, Seiten 158-164, XP009117569 & 6TH INTERNATIONAL SYMPOSIUM ON YERSINIA; ROME, ITALY; SEPTEMBER 26-28, 1994
- IUSHCHUK N D ET AL: "[Laboratory diagnosis of Yersinia infection and its improvement]" ZHURNAL MIKROBIOLOGII, EPIDEMIOLOGII, I IMMUNOBIOLOGII 2007 MAY-JUN, Nr. 3, Mai 2007 (2007-05), Seiten 61-66, XP002529754 ISSN: 0372-9311
- DEVDARIANI Z L ET AL: "Enzyme immunoassay system for identification of typical and atypical strains of plague microbe" MEDICINSKAA PARAZITOLOGIA I PARAZITARNYE BOLEZNI, MEDICINA, MOSCOW, RU, Nr. 1, 1. Januar 1997 (1997-01-01), Seiten 31-33, XP009117087 ISSN: 0025-8326
- ROBINS-BROWNE ET AL: "Yersinia Species" 1. Januar 2003 (2003-01-01), INTERNATIONAL HANDBOOK OF FOODBORNE PATHOGENS,, PAGE(S) 323 - 356 , XP009117353 Seite 333 - Seite 345
- GOMES-SOLECKI MARIA J C ET AL: "LcrV capture enzyme-linked immunosorbent assay for detection of Yersinia pestis from human samples." CLINICAL AND DIAGNOSTIC LABORATORY IMMUNOLOGY FEB 2005, Bd. 12, Nr. 2, Februar 2005 (2005-02), Seiten 339-346, XP002529755 ISSN: 1071-412X
- HEESEMANN J ET AL: "Analysis of the class-specific immune response to Yersinia enterocolitica virulence-associated antigens in oro-gastrically infected rabbits" MICROBIAL PATHOGENESIS, ACADEMIC PRESS LIMITED, NEW YORK, NY, US, Bd. 5, Nr. 6, 1. Dezember 1988 (1988-12-01), Seiten 437-447, XP023312563 ISSN: 0882-4010 [gefunden am 1988-12-01]
- HENSEL ANDREAS ET AL: "Zur Prävalenz von Anti-Yersinia-Outer-Protein-Antikörpern bei Schlachtschweinen in Bayern = The prevalence of anti-yersinia outer protein antibodies in Bavarian slaughter pigs" BERLINER UND MUENCHENER TIERAERZTLICHE WOCHENSCHRIFT, PAUL PAREY, BERLIN, DE, Bd. 117, Nr. 1-2, 1. Januar 2004 (2004-01-01), Seiten 30-38, XP009133044 ISSN: 0005-9366
- BENNER G E ET AL: "Immune response to Yersinia outer proteins and other Yersinia pestis antigens after experimental plague infection in mice." INFECTION AND IMMUNITY APR 1999 LNKD- PUBMED:10085037, Bd. 67, Nr. 4, April 1999 (1999-04), Seiten 1922-1928, XP002582458 ISSN: 0019-9567
- CHEN Z ET AL: "Quorum sensing affects virulence-associated proteins F1, LcrV, KatY and pH6 etc. of Yersinia pestis as revealed by protein microarray-based antibody profiling", MICROBES AND INFECTION, ELSEVIER, PARIS, FR, vol. 8, no. 9-10, 1 August 2006 (2006-08-01), pages 2501-2508, XP028072089, ISSN: 1286-4579, DOI: 10.1016/J.MICINF.2006.06.007 [retrieved on 2006-08-01]
- LI BEI ET AL: "Protein microarray for profiling antibody responses to Yersinia pestis live vaccine", INFECTION AND IMMUNITY, AMERICAN SOCIETY FOR MICROBIOLOGY, US, vol. 73, no. 6, 1 June 2005 (2005-06-01), pages 3734-3739, XP002441454, ISSN: 0019-9567, DOI: 10.1128/IAI.73.6.3734-3739.2005
- Niefnecker (2009): "Identifizierung von virulenzassoziierten Antigenen bei enteropathogenen Yersinien und Campylobacter jejuni", Dissertation, LMU
- Mikrogen (2005): recomLine Yersinia IgG recomLine Yersinia IgA ¬IgM| - Gebrauchsinformation

### SEQUENCE LISTING

<110> Mikrogen molekularbiologische Entwicklungs - GmbH
<120> Vorrichtung zum serologischen Nachweis der humanen Yersinien-Infektionen und/oder deren Folgeerkrankungen sowie Verwendung der Proteine MyfA und PsaA von Y. enterocolitica und Y. pseudotuberculosis als rekombinante Antigene
<130> MyfA,PsaA
<160> 24
<170> PatentIn version 3.5
<210> 1
   <211> 306
   <212> PRT
   <213> Yersinia
<400> 1
   Met Thr Ile Asn Ile Lys Thr Asp Ser Pro Ile Ile Thr Thr Gly Ser
   1               5                   10                  15
   Gln Ile Asp Ala Ile Thr Thr Glu Thr Val Gly Gln Ser Gly Glu Val
               20                  25                  30
   Lys Lys Thr Glu Asp Thr Arg His Glu Ala Gln Ala Ile Lys Ser Ser
           35                  40                  45
   Glu Ala Ser Leu Ser Arg Ser Gln Val Pro Glu Leu Ile Lys Pro Ser
       50                  55                  60
   Gln Gly Ile Asn Val Ala Leu Leu Ser Lys Ser Gln Gly Asp Leu Asn
   65                  70                  75                  80
   Gly Thr Leu Ser Ile Leu Leu Leu Leu Leu Glu Leu Ala Arg Lys Ala
                   85                  90                  95
   Arg Glu Met Gly Leu Gln Gln Arg Asp Ile Glu Asn Lys Ala Ala Ile
               100                 105                 110
   Thr Ala Gln Lys Glu Gln Val Ala Glu Met Val Ser Gly Ala Lys Leu
           115                 120                 125
   Met Ile Ala Met Ala Val Val Ser Gly Ile Met Ala Ala Thr Ser Thr
       130                 135                 140
   Val Ala Ser Ala Phe Ser Ile Ala Lys Glu Val Lys Ile Val Lys Gln
   145                 150                 155                 160
   Glu Gln Ile Leu Asn Ser Asn Ile Ala Gly Arg Asp Gln Leu Ile Asp
   165 170 175
   Thr Lys Leu Gln Gln Met Ser Asn Thr Ser Asp Lys Ala Val Ser Arg
   180 185 190
   Glu Asp Ile Gly Arg Ile Trp Lys Pro Glu Gln Val Ala Asp Gln Asn
   195 200 205
   Lys Leu Ala Leu Leu Asp Lys Glu Phe Arg Met Thr Asp Ser Lys Ala
   210 215 220
   Asn Ala Phe Asn Ala Ala Thr Gln Pro Leu Gly Gln Met Ala Asn Ser
   225 230 235 240
   Ala Ile Gln Val His Arg Gly Tyr Ser Gln Ala Glu Val Lys Glu Lys
   245 250 255
   Glu Val Asn Ala Ser Ile Ala Ala Asn Glu Lys Gln Lys Ala Glu Glu
   260 265 270
   Ala Met Asn Tyr Asn Asp Asn Phe Met Lys Asp Val Leu Arg Leu Ile
   275 280 285
   Glu Gln Tyr Val Ser Ser His Thr His Ala Met Lys Ala Ala Phe Gly
   290 295 300
   Val Val
   305
<210> 2
   <211> 468
   <212> PRT
   <213> Yersinia
<400> 2
   Met Asn Leu Ser Leu Ser Asp Leu His Arg Gln Val Ser Arg Leu Val
   1 5 10 15
   Gln Gln Glu Ser Gly Asp Cys Thr Gly Lys Leu Arg Gly Asn Val Ala
   20 25 30
   Ala Asn Lys Glu Thr Thr Phe Gln Gly Leu Thr Ile Ala Ser Gly Ala
   35 40 45
   Arg Glu Ser Glu Lys Val Phe Ala Gln Thr Val Leu Ser His Val Ala
           50                  55                  60
   Asn Ile Val Leu Thr Gln Glu Asp Thr Ala Lys Leu Leu Gln Ser Thr
   65                  70                  75                  80
   Val Lys His Asn Leu Asn Asn Tyr Glu Leu Arg Ser Val Gly Asn Gly
                   85                  90                  95
   Asn Ser Val Leu Val Ser Leu Arg Ser Asp Gln Met Thr Leu Gln Asp
               100                 105                 110
   Ala Lys Val Leu Leu Glu Ala Ala Leu Arg Gln Glu Ser Gly Ala Arg
           115                 120                 125
   Gly His Val Ser Ser His Ser His Ser Val Leu His Ala Pro Gly Thr
       130                 135                 140
   Pro Val Arg Glu Gly Leu Arg Ser His Leu Asp Pro Arg Thr Pro Pro
   145                 150                 155                 160
   Leu Pro Pro Arg Glu Arg Pro His Thr Ser Gly His His Gly Ala Gly
                   165                 170                 175
   Glu Ala Arg Ala Thr Ala Pro Ser Thr Val Ser Pro Tyr Gly Pro Glu
               180                 185                 190
   Ala Arg Ala Glu Leu Ser Ser Arg Leu Thr Thr Leu Arg Asn Thr Leu
           195                 200                 205
   Ala Pro Ala Thr Asn Asp Pro Arg Tyr Leu Gln Ala Cys Gly Gly Glu
       210                 215                 220
   Lys Leu Asn Arg Phe Arg Asp Ile Gln Cys Cys Arg Gln Thr Ala Val
   225                 230                 235                 240
   Arg Ala Asp Leu Asn Ala Asn Tyr Ile Gln Val Gly Asn Thr Arg Thr
                   245                 250                 255
   Ile Ala Cys Gln Tyr Pro Leu Gln Ser Gln Leu Glu Ser His Phe Arg
               260                 265                 270
   Met Leu Ala Glu Asn Arg Thr Pro Val Leu Ala Val Leu Ala Ser Ser
           275                 280                 285
   Ser Glu Ile Ala Asn Gln Arg Phe Gly Met Pro Asp Tyr Phe Arg Gln
       290                 295                 300
   Ser Gly Thr Tyr Gly Ser Ile Thr Val Glu Ser Lys Met Thr Gln Gln
   305 310 315 320
   Val Gly Leu Gly Asp Gly Ile Met Ala Asp Met Tyr Thr Leu Thr Ile
   325 330 335
   Arg Glu Ala Gly Gln Lys Thr Ile Ser Val Pro Val Val His Val Gly
   340 345 350
   Asn Trp Pro Asp Gln Thr Ala Val Ser Ser Glu Val Thr Lys Ala Leu
   355 360 365
   Ala Ser Leu Val Asp Gln Thr Ala Glu Thr Lys Arg Asn Met Tyr Glu
   370 375 380
   Ser Lys Gly Ser Ser Ala Val Ala Asp Asp Ser Lys Leu Arg Pro Val
   385 390 395 400
   Ile His Cys Arg Ala Gly Val Gly Arg Thr Ala Gln Leu Ile Gly Ala
   405 410 415
   Met Cys Met Asn Asp Ser Arg Asn Ser Gln Leu Ser Val Glu Asp Met
   420 425 430
   Val Ser Gln Met Arg Val Gln Arg Asn Gly Ile Met Val Gln Lys Asp
   435 440 445
   Glu Gln Leu Asp Val Leu Ile Lys Leu Ala Glu Gly Gln Gly Arg Pro
   450 455 460
   Leu Leu Asn Ser
   465
<210> 3
   <211> 293
   <212> PRT
   <213> Yersinia
<400> 3
   Met Thr Thr Leu His Asn Ile Ser Tyr Gly Asn Thr Thr Leu Arg Asn
   1 5 10 15
   Glu His Pro Glu Thr Ala Ser Ser Gln Ile Val Asn Gln Thr Leu Gly
   20 25 30
   Gln Phe Arg Gly Glu Ser Val Gln Ile Val Ser Gly Thr Leu Gln Ser
   35 40 45
   Ile Ala Asp Met Ala Glu Glu Val Thr Phe Val Phe Ser Glu Arg Lys
   50 55 60
   Glu Leu Ser Leu Asp Lys Arg Lys Leu Ser Asp Ser Gln Ala Arg Val
   65 70 75 80
   Ser Asp Val Glu Glu Gln Val Asn Gln Tyr Leu Ser Lys Val Pro Glu
   85 90 95
   Leu Glu Gln Lys Gln Asn Val Ser Glu Leu Leu Ser Leu Leu Ser Asn
   100 105 110
   Ser Pro Asn Ile Ser Leu Ser Gln Leu Lys Ala Tyr Leu Glu Gly Lys
   115 120 125
   Ser Glu Glu Pro Ser Glu Gln Phe Lys Met Leu Cys Gly Leu Arg Asp
   130 135 140
   Ala Leu Lys Gly Arg Pro Glu Leu Ala His Leu Ser His Leu Val Glu
   145 150 155 160
   Gln Ala Leu Val Ser Met Ala Glu Glu Gln Gly Glu Ala Ile Val Leu
   165 170 175
   Gly Ala Arg Ile Thr Pro Glu Ala Tyr Arg Glu Ser Gln Ser Ser Val
   180 185 190
   Asn Pro Leu Gln Pro Leu Arg Asp Thr Tyr Arg Asp Ala Val Met Gly
   195 200 205
   Tyr Gln Gly Ile Tyr Ala Ile Trp Ser Asp Leu Gln Lys Arg Phe Pro
   210 215 220
   Asn Gly Asp Ile Asp Ser Val Ile Leu Phe Leu Gln Lys Ala Leu Ser
   225 230 235 240
   Ala Asp Leu Gln Ser Gln Gln Ser Gly Ser Gly Arg Glu Lys Leu Gly
   245 250 255
   Ile Val Ile Ser Asp Leu Gln Lys Leu Lys Glu Phe Gly Ser Val Ser
   260 265 270
   Asp Gln Val Lys Gly Phe Trp Gln Phe Phe Ser Glu Gly Lys Thr Asn
   275 280 285
   Gly Val Arg Pro Phe
   290
<210> 4
   <211> 220
   <212> PRT
   <213> Yersinia
<400> 4
   Met Pro Lys Ile Ser Ser Phe Ile Ser Thr Ser Leu Pro Leu Pro Thr
   1 5 10 15
   Ser Val Ser Gly Ser Ser Ser Val Gly Glu Met Ser Gly Arg Ser Val
   20 25 30
   Ser Gln Gln Lys Ser Glu Gln Tyr Ala Asn Asn Leu Ala Gly Arg Thr
   35 40 45
   Glu Ser Pro Gln Gly Ser Ser Leu Ala Ser Arg Ile Thr Glu Lys Leu
   50 55 60
   Ser Ser Met Ala Arg Ser Ala Ile Glu Phe Ile Lys Arg Met Phe Ser
   65 70 75 80
   Glu Gly Ser His Lys Pro Val Val Thr Pro Ala Pro Thr Pro Ala Gln
   85 90 95
   Met Pro Ser Pro Thr Ser Phe Ser Asp Ser Ile Lys Gln Leu Ala Ala
   100 105 110
   Glu Thr Leu Pro Lys Tyr Ile Gln Gln Leu Ser Ser Leu Asp Ala Glu
   115 120 125
   Thr Leu Gln Lys Asn His Asp Gln Phe Ala Thr Gly Ser Gly Pro Leu
   130 135 140
   Arg Gly Ser Ile Thr Gln Cys Gln Gly Leu Met Gln Phe Cys Gly Gly
   145 150 155 160
   Glu Leu Gln Ala Glu Ala Ser Ala Ile Leu Asn Thr Pro Val Cys Gly
   165 170 175
   Ile Pro Phe Ser Gln Trp Gly Thr Ile Gly Gly Ala Ala Ser Ala Tyr
   180 185 190
   Val Ala Ser Gly Val Asp Leu Thr Gln Ala Ala Asn Glu Leu Lys Gly
   195 200 205
   Leu Ala Gln Gln Met His Gln Leu Leu Ser Leu Met
   210 215 220
<210> 5
   <211> 505
   <212> PRT
   <213> Yersinia
<400> 5
   Met Phe Ile Asn Pro Arg Asn Val Ser Asn Thr Phe Leu Gln Glu Pro
   1 5 10 15
   Leu Arg His Ser Ser Asp Leu Thr Glu Ile Pro Val Glu Ala Glu Asn
   20 25 30
   Val Lys Ser Lys Thr Glu Tyr Tyr Asn Ala Trp Ser Glu Trp Glu Arg
   35 40 45
   Asn Ala Pro Pro Gly Asn Gly Glu Gln Arg Glu Met Ala Val Ser Arg
   50 55 60
   Leu Arg Asp Cys Leu Asp Arg Gln Ala His Glu Leu Glu Leu Asn Asn
   65 70 75 80
   Leu Gly Leu Ser Ser Leu Pro Glu Leu Pro Pro His Leu Glu Arg Leu
   85 90 95
   Val Ala Ser Cys Asn Ser Leu Thr Glu Leu Pro Glu Leu Pro Gln Ser
   100 105 110
   Leu Lys Ser Leu Glu Val Tyr Glu Asn Asn Leu Lys Ala Leu Pro Asp
   115 120 125
   Leu Pro Pro Leu Leu Val Asp Leu Arg Val Phe Asn Asn Gln Leu Glu
   130 135 140
   Glu Leu Pro Glu Leu Gln Asn Leu Pro Phe Leu Thr Glu Ile Tyr Ala
   145 150 155 160
   Asn Asn Asn Ser Leu Lys Thr Leu Pro Asp Leu Pro Pro Ser Leu Val
   165 170 175
   Asp Leu Asn Val Arg Glu Asn Tyr Leu Thr Ala Leu Pro Glu Leu Pro
   180 185 190
   Gln Ser Leu Ile Phe Leu Asp Ile Ser Asp Asn Ile Leu Ser Gly Leu
   195 200 205
   Ser Glu Leu Pro Pro Asn Leu Ser Cys Leu Asp Ala Ser Arg Asn Gly
   210 215 220
   Ile Arg Ser Leu Cys Asp Leu Pro Pro Ser Leu Val Tyr Leu Asp Val
   225 230 235 240
   Arg Asp Asn Gln Leu Ile Glu Leu Pro Ala Leu Pro Ser Gly Leu Glu
   245 250 255
   Arg Leu Ile Ala Ser Phe Asn His Leu Ala Glu Leu Pro Glu Leu Pro
   260 265 270
   Pro Asn Leu Tyr Tyr Leu Asp Ala Ser Arg Asn Glu Ile Ser Ser Leu
   275 280 285
   Cys Asp Leu Pro Pro Ser Leu Val Asp Leu Asn Val Arg Lys Asn Gln
   290 295 300
   Leu Ile Glu Leu Pro Ala Leu Pro Pro Asp Leu Glu Arg Leu Ile Ala
   305 310 315 320
   Ser Phe Asn His Leu Ala Glu Leu Pro Glu Leu Pro Pro Asn Leu Ser
   325 330 335
   Tyr Leu Asp Ala Ser Arg Asn Glu Ile Ser Ser Leu Cys Asp Leu Pro
   340 345 350
   Pro Ser Leu Val Asp Leu Asn Val Arg Lys Asn Gln Leu Ile Glu Leu
   355 360 365
   Pro Ala Leu Pro Pro Asp Leu Glu Arg Leu Ile Ala Ser Phe Asn His
   370 375 380
   Leu Ala Glu Leu Pro Glu Leu Pro Pro Asn Leu Ser Tyr Leu Asp Ala
   385 390 395 400
   Ser Arg Asn Glu Ile Ser Ser Leu Cys Asp Leu Pro Pro Ser Leu Val
   405 410 415
   Glu Leu Asp Val Arg Asp Asn Gln Leu Ile Glu Leu Pro Ala Leu Pro
   420 425 430
   Pro His Leu Glu Arg Leu Ile Ala Ser Leu Asn His Leu Ala Glu Val
   435 440 445
   Pro Glu Leu Pro Gln Asn Leu Lys Gln Leu His Val Glu His Asn Ala
   450 455 460
   Leu Arg Glu Phe Pro Asp Ile Pro Glu Ser Val Glu Asp Leu Arg Met
   465 470 475 480
   Asp Ser Glu Arg Val Ile Asp Pro Tyr Glu Phe Ala His Glu Thr Ile
   485 490 495
   Asp Lys Leu Glu Asp Asp Val Phe Glu
   500 505
<210> 6
   <211> 333
   <212> PRT
   <213> Yersinia
<400> 6
   Met Ile Arg Ala Tyr Glu Gln Asn Pro Gln His Phe Ile Glu Asp Leu
   1 5 10 15
   Glu Lys Val Arg Val Glu Gln Leu Thr Gly His Gly Ser Ser Val Leu
   20 25 30
   Glu Glu Leu Val Gln Leu Val Lys Asp Lys Lys Ile Asp Ile Ser Ile
   35 40 45
   Lys Tyr Asp Pro Lys Lys Asp Ser Glu Val Phe Ala Glu Arg Val Ile
   50 55 60
   Thr Asp Asp Ile Glu Leu Leu Lys Lys Ile Leu Ala Tyr Phe Leu Pro
   65 70 75 80
   Glu Asp Ala Ile Leu Lys Gly Gly His Tyr Asp Asn Gln Leu Gln Asn
   85 90 95
   Gly Ile Lys Arg Val Lys Glu Phe Leu Glu Ser Ser Pro Asn Thr Gln
   100 105 110
   Trp Glu Leu Arg Ala Phe Met Ala Val Met His Phe Ser Leu Thr Ala
   115 120 125
   Asp Arg Ile Asp Asp Asp Ile Leu Lys Val Ile Val Asp Ser Met Asn
       130                 135                 140
   His His Gly Asp Ala Arg Ser Lys Leu Arg Glu Glu Leu Ala Glu Leu
   145                 150                 155                 160
   Thr Ala Glu Leu Lys Ile Tyr Ser Val Ile Gln Ala Glu Ile Asn Lys
                   165                 170                 175
   His Leu Ser Ser Ser Gly Thr Ile Asn Ile His Glu Lys Ser Ile Asn
               180                 185                 190
   Leu Met Asp Lys Asn Leu Tyr Gly Tyr Thr Asp Glu Glu Ile Phe Lys
           195                 200                 205
   Ala Ser Ala Glu Tyr Lys Ile Leu Lys Lys Met Pro Gln Thr Thr Ile
       210                 215                 220
   Lys Asp Asp Glu Leu His Glu Val Gly Val Ile Ala Gly Ala Glu Lys
   225                 230                 235                 240
   Gln Ile Val Ser Ile Lys Asn Phe Leu Glu Ser Glu Asn Lys Arg Thr
                   245                 250                 255
   Gly Ala Leu Gly Asn Leu Lys Asp Ser Tyr Ser Tyr Asn Lys Asp Asn
               260                 265                 270
   Asn Glu Leu Ser His Phe Ala Thr Ala Cys Ser Asp Lys Ser Arg Pro
           275                 280                 285
   Leu Asn Asp Leu Val Ser Gln Lys Thr Thr Gln Leu Ser Asp Ile Thr
       290                 295                 300
   Ser Arg Phe Asn Ser Ala Ile Glu Ala Leu Asn Arg Phe Ile Gln Lys
   305                 310                 315                 320
   Tyr Asp Ser Val Met Gln Arg Leu Leu Asp Asp Thr Arg
                   325                 330
<210> 7
   <211> 131
   <212> PRT
   <213> Yersinia
<400> 7
   Met Glu Pro Thr Val Ile Asn Ser Lys Asp Ile Ser Ala Thr Lys Thr
   1               5                   10                  15
   Val Lys Glu Gly Gly Ser Phe Ser Val Glu Phe Lys Ala Thr Glu Asn
   20 25 30
   Glu Ile Val Ser Gly Lys Leu Asp Ala Asp Thr Pro Ala Phe His Leu
   35 40 45
   Val Met Ser Asp Ser Gly Glu His Lys Gly Trp Asn Val Arg Pro Thr
   50 55 60
   Gly Ala Ser Glu Gly Gly Gln Met Val Ser Ala Asp Gly Thr Arg Val
   65 70 75 80
   Asp Leu His Thr Asn Glu Leu Ser Trp Asp Asn Asp His Trp Trp Ile
   85 90 95
   Asp Asp Gly Ser Glu Arg Val Glu Ala Thr Phe Phe Leu Ala Ala Gly
   100 105 110
   Asp Glu Val Lys Ala Gly Glu Tyr Gln Phe Thr Gly Arg Val Glu Glu
   115 120 125
   Tyr Val Glu
   130
<210> 8
   <211> 133
   <212> PRT
   <213> Yersinia
<400> 8
   Met Ser Thr Val Ile Asn Ser Lys Asp Val Ser Gly Glu Val Thr Val
   1 5 10 15
   Lys Gln Gly Asn Thr Phe His Val Asp Phe Ala Pro Asn Thr Gly Glu
   20 25 30
   Ile Phe Ala Gly Lys Gln Pro Gly Asp Val Thr Met Phe Thr Leu Thr
   35 40 45
   Met Gly Asp Thr Ala Pro His Gly Gly Trp Arg Leu Ile Pro Thr Gly
   50 55 60
   Asp Ser Lys Gly Gly Tyr Met Ile Ser Ala Asp Gly Asp Tyr Val Gly
   65 70 75 80
   Leu Tyr Ser Tyr Met Met Ser Trp Val Gly Ile Asp Asn Asn Trp Tyr
   85 90 95
   Ile Asn Asp Asp Ser Pro Lys Asp Ile Lys Asp His Leu Tyr Val Lys
   100 105 110
   Ala Gly Thr Val Leu Lys Pro Thr Thr Tyr Lys Phe Thr Gly Arg Val
   115 120 125
   Glu Glu Tyr Val Phe
   130
<210> 9
   <211> 396
   <212> DNA
   <213> Yersinia
<400> 9
   atggaaccga ctgttattaa tagtaaagac atctctgcaa caaaaactgt taaagaggga 60
   ggttcgttct cagttgaatt caaggccact gaaaacgaga ttgtgtcagg caaattggat 120
   gcagatacac ctgccttcca tctggtaatg tcggactcag gggaacataa aggttggaat 180
   gttcggccta ccggtgcatc tgagggagga cagatggttt ctgcagatgg taccagagtt 240
   gacttacata caaatgagct atcgtgggat aacgaccact ggtggataga tgacggttct 300
   gagcgtgtgg aagcgacttt ctttcttgct gctggcgacg aggttaaagc aggtgaatat 360
   cagttcactg ggcgtgttga ggaatatgtc gagtaa 396
<210> 10
   <211> 402
   <212> DNA
   <213> Yersinia
<400> 10
   atgtctactg tcattaactc caaggatgtt tctggtgagg tgactgtcaa gcagggaaac 60
   acattccacg tcgattttgc gcctaacaca ggagagattt ttgcgggtaa acagccgggt 120
   gatgtcacta tgtttacgct aactatgggt gatactgcac cacacggtgg ttggcgtttg 180
   attccaacag gggactcaaa aggtggatat atgatcagcg ccgatggtga ctatgttggt 240
   ttatacagtt atatgatgtc atgggtaggt atagataata actggtatat aaatgatgac 300
   tctcctaaag atataaaaga tcatctgtac gttaaggcag ggactgtcct taaaccaacg 360
   acttataaat tcacggggcg tgttgaagag tatgtatttt aa 402
<210> 11
   <211> 159
   <212> PRT
   <213> Yersinia
<400> 11
   Met Asn Met Lys Lys Phe Val Lys Lys Pro Leu Ala Ile Ala Val Leu
   1               5                   10                  15
   Met Leu Ala Ser Gly Gly Met Val Asn Met Val His Ala Glu Pro Thr
               20                  25                  30
   Val Ile Asn Ser Lys Asp Ile Ser Ala Thr Lys Thr Val Lys Glu Gly
           35                  40                  45
   Gly Ser Phe Ser Val Glu Phe Lys Ala Thr Glu Asn Glu Ile Val Ser
       50                  55                  60
   Gly Lys Leu Asp Ala Asp Thr Pro Ala Phe His Leu Val Met Ser Asp
   65                  70                  75                  80
   Ser Gly Glu His Lys Gly Trp Asn Val Arg Pro Thr Gly Ala Ser Glu
                   85                  90                  95
   Gly Gly Gln Met Val Ser Ala Asp Gly Thr Arg Val Asp Leu His Thr
               100                 105                 110
   Asn Glu Leu Ser Trp Asp Asn Asp His Trp Trp Ile Asp Asp Gly Ser
           115                 120                 125
   Glu Arg Val Glu Ala Thr Phe Phe Leu Ala Ala Gly Asp Glu Val Lys
       130                 135                 140
   Ala Gly Glu Tyr Gln Phe Thr Gly Arg Val Glu Glu Tyr Val Glu
   145                 150                 155
<210> 12
   <211> 158
   <212> PRT
   <213> Yersinia
<400> 12
   Met Lys Met Lys Cys Phe Ala Lys Asn Ala Leu Ala Val Thr Thr Leu
   1               5                   10                  15
   Met Ile Ala Ala Cys Gly Met Ala Asn Ala Ser Thr Val Ile Asn Ser
               20                  25                  30
   Lys Asp Val Ser Gly Glu Val Thr Val Lys Gln Gly Asn Thr Phe His
           35                  40                  45
   Val Asp Phe Ala Pro Asn Thr Gly Glu Ile Phe Ala Gly Lys Gln Pro
   50 55 60
   Gly Asp Val Thr Met Phe Thr Leu Thr Met Gly Asp Thr Ala Pro His
   65 70 75 80
   Gly Gly Trp Arg Leu Ile Pro Thr Gly Asp Ser Lys Gly Gly Tyr Met
   85 90 95
   Ile Ser Ala Asp Gly Asp Tyr Val Gly Leu Tyr Ser Tyr Met Met Ser
   100 105 110
   Trp Val Gly Ile Asp Asn Asn Trp Tyr Ile Asn Asp Asp Ser Pro Lys
   115 120 125
   Asp Ile Lys Asp His Leu Tyr Val Lys Ala Gly Thr Val Leu Lys Pro
   130 135 140
   Thr Thr Tyr Lys Phe Thr Gly Arg Val Glu Glu Tyr Val Phe
   145 150 155
<210> 13
   <211> 30
   <212> DNA
   <213> Artificial Sequence
<220>
   <223> primer
<400> 13
   gtaattccat atgaatatga aaaaatttgt 30
<210> 14
   <211> 27
   <212> DNA
   <213> Artificial Sequence
<220>
   <223> primer
<400> 14
   gtaatcccat atggcaacaa aaactgt 27
<210> 15
   <211> 24
   <212> DNA
   <213> Artificial Sequence
<220>
   <223> primer
<400> 15
   ttactcgagt tcacctgctt taac 24
<210> 16 <211> 28
   <212> DNA
   <213> Artificial Sequence
<220>
   <223> primer
<400> 16
   atctactcga gctcgacata ttcctcaa 28
<210> 17
   <211> 38
   <212> PRT
   <213> Artificial Sequence
<220>
   <223> primer'
<400> 17 <210> 18
   <211> 32
   <212> DNA
   <213> Artificial Sequence
<220>
   <223> primer <
400> 18
   atggatcctt actcgacata ttcctcaaca cg 32
<210> 19
   <211> 33
   <212> DNA
   <213> Artificial Sequence
<220>
   <223> primer
<400> 19
   gccatatgtc tactgtcatt aactccaagg atg 33
<210> 20
   <211> 34
   <212> DNA
   <213> Artificial Sequence
<220>
   <223> prlmer
<400> 20
   caggatcctt aaaatacata ctcttcaaca cgcc 34
<210> 21
   <211> 396
   <212> DNA
   <213> Yersinia
<400> 21
   atggaaccga ctgttattaa tagtaaagac atctctgcaa caaaaactgt taaagaggga 60
   ggttcgttct cagttgaatt caaggccact gaaaacgaga ttgtgtcagg caaattggat 120
   gcagatacac ctgccttcca tctggtaatg tcggactcag gggaacataa aggttggaat 180
   gttcggccta ccggtgcatc tgagggagga cagatggttt ctgcagatgg taccagagtt 240
   gacttacata caaatgagct atcgtgggat aacgaccact ggtggataga tgacggttct 300
   gagcgtgtgg aagcgacttt ctttcttgct gctggcgacg aggttaaagc aggtgaatat 360
   cagttcactg ggcgtgttga ggaatatgtc gagtaa 396
<210> 22
   <211> 402
   <212> DNA
   <213> Yersinia
<400> 22
   atgtctactg tcattaactc caaggatgtt tctggtgagg tgactgtcaa gcagggaaac 60
   acattccacg tcgattttgc gcctaacaca ggagagattt ttgcgggtaa acagccgggt 120
   gatgtcacta tgtttacgct aactatgggt gatactgcac cacacggtgg ttggcgtttg 180
   attccaacag gggactcaaa aggtggatat atgatcagcg ccgatggtga ctatgttggt 240
   ttatacagtt atatgatgtc atgggtaggt atagataata actggtatat aaatgatgac 300
   tctcctaaag atataaaaga tcatctgtac gttaaggcag ggactgtcct taaaccaacg 360
   acttataaat tcacggggcg tgttgaagag tatgtatttt aa 402
<210> 23
   <211> 131
   <212> PRT
   <213> Yersinia
<400> 23
   Met Glu Pro Thr Val Ile Asn Ser Lys Asp Ile Ser Ala Thr Lys Thr
   1 5 10 15
   Val Lys Glu Gly Gly Ser Phe Ser Val Glu Phe Lys Ala Thr Glu Asn
   20 25 30
   Glu Ile Val Ser Gly Lys Leu Asp Ala Asp Thr Pro Ala Phe His Leu
   35 40 45
   Val Met Ser Asp Ser Gly Glu His Lys Gly Trp Asn Val Arg Pro Thr
   50 55 60
   Gly Ala Ser Glu Gly Gly Gln Met Val Ser Ala Asp Gly Thr Arg Val
   65 70 75 80
   Asp Leu His Thr Asn Glu Leu Ser Trp Asp Asn Asp His Trp Trp Ile
   85 90 95
   Asp Asp Gly Ser Glu Arg Val Glu Ala Thr Phe Phe Leu Ala Ala Gly
   100 105 110
   Asp Glu Val Lys Ala Gly Glu Tyr Gln Phe Thr Gly Arg Val Glu Glu
   115 120 125
   Tyr Val Glu
   130
<210> 24
   <211> 133
   <212> PRT
   <213> Yersinia
<400> 24
   Met Ser Thr Val Ile Asn Ser Lys Asp Val Ser Gly Glu Val Thr Val
   1 5 10 15
   Lys Gln Gly Asn Thr Phe His Val Asp Phe Ala Pro Asn Thr Gly Glu
   20 25 30
   Ile Phe Ala Gly Lys Gln Pro Gly Asp Val Thr Met Phe Thr Leu Thr
   35 40 45
   Met Gly Asp Thr Ala Pro His Gly Gly Trp Arg Leu Ile Pro Thr Gly
   50 55 60
   Asp Ser Lys Gly Gly Tyr Met Ile Ser Ala Asp Gly Asp Tyr Val Gly
   65                  70                  75                  80
   Leu Tyr Ser Tyr Met Met Ser Trp Val Gly Ile Asp Asn Asn Trp Tyr
                   85                  90                  95
   Ile Asn Asp Asp Ser Pro Lys Asp Ile Lys Asp His Leu Tyr Val Lys
               100                 105                 110
   Ala Gly Thr Val Leu Lys Pro Thr Thr Tyr Lys Phe Thr Gly Arg Val
           115                 120                 125
   Glu Glu Tyr Val Phe
       130

## Beschreibung

Das Genus *Yersinia* umfasst drei humanpathogene *Yersinia* spp., *Y. pestis, Y. pseudotuberculosis* und *Y. enterocolitica* und acht weitere Species wie *Y. aldovae, Y. bercovieri, Y. frederiksenii, Y. intermedia, Y. kristensenii, Y. moolaretti, Y. rohdei* und *Y. ruckeri*, die eher als opportunistische Krankheitserreger bei Wunden und Sepsis beim Menschen eine Rolle spielen. *Y. pestis* ist der Erreger der Pest und kann durch Flöhe von dem natürlichen Nagetier-Reservoir auf den Menschen übertragen werden. *Y. enterocolitica* und *Y. pseudotuberculosis* treten weltweit in den gemäßigten bis subtropischen Klimazonen bei Wild- und Nutztieren auf. In Deutschland ist die durch *Y. enterocolitica* und *Y. pseudotuberculosis* hervorgerufene Darmerkrankung, die sog. Yersiniose, nach der Salmonellose (*Salmonella enterica*) und Campylobacter-Enteritis (*Campylobacter jejuni*) die dritthäufigste bakterielle Enteritiserkrankung, die dem Robert Koch-Institut (RKI) Berlin jährlich gemeldet wird. Im Jahr 2007 wurden in Deutschland 4.987 und im Jahr 2006 5.162 durch Yersinien bedingte Erkrankungen gemäß RKI-Falldefinition an das Robert Koch -Institut übermittelt. Die höchste altersspezifische Inzidenz wurde bei Kleinkindern im Alter von 1 bis 4 Jahren beobachtet (RKI). Die tatsächliche, Häufigkeit dieser Erkrankungen ist allerdings nicht genau bekannt - viele Erkrankte suchen bei subklinischen bzw. bei leichten und kurzen Krankheitsverläufen keinen Arzt auf, viele Erkrankungen werden ätiologisch nicht geklärt und nicht alle diagnostizierten Erkrankungsfälle werden gemeldet. Schätzungsweise bis zu 43% der Blutspender in Deutschland und 31% der Blutspender in Finnland haben spezifische Antikörper gegen Yersinien (getestet mit Hilfe von Enzym-Immunoassays bzw. Immunoblots). Dieses Ergebnis lässt eine unerwartet hohe Prävalenz von durchgemachten Yersiniosen in Industrieländern mit hohen Hygienestandards vermuten.

Die enteropathogenen *Y. enterocolitica*- und *Y. pseudotuberculosis* -Erreger kommen bei Schweinen, Schafen, Rindern und Geflügel vor und gelangen hauptsächlich durch den Schlachtprozess in Lebensmittel (z.B. rohes Schweinefleisch) und Trinkwasser. Darüber hinaus sind Übertragungen auf Haustiere beschrieben. Auch konnte festgestellt werden, dass bis zu 100% der Wildschweine *Yersinia enterocolitica* im Nasenrachenraum tragen. Yersinien besitzen die Fähigkeit, sich bei niedrigen Temperaturen (> 4°C) zu vermehren. In den Industrieländern stellt die Vermehrung in gekühlten Lebensmitteln die epidemiologisch wichtigste Verbreitungsform von *Y. enterocolitica* und *Y. pseudotuberculosis* dar.

Die genaue Infektionsdosis ist nicht bekannt (vermutlich >10000 Bakterien). Die Inkubationszeit beträgt zwei bis fünf Tage nach der Ansteckung. Enteropathogene *Y. enterocolitica* und *Y. pseudotuberculosis* -Stämme siedeln sich meist im darmassoziierten lymphatischen Gewebe des terminalen Ileums, in den so genannten Peyer'schen Plaques (zusammenhängende Ansammlung von Lymphfollikeln), an. Nach Ansiedlung überwinden die Yersinien über sog. M-Zellen in der Follikel-assoziierten Epithelzellschicht der Peyer'schen Plaques die gastrointestinale Barriere des Organismus und breiten sich über die abfließenden Lymphgefäße in den mesenterialen Lymphknoten aus. Durch *Y. enterocolitica* werden nachfolgend weitere Darmbereiche befallen und geschädigt, was die typischen Gewebsverletzungen des Dünn- und Dickdarms bei Yersiniose-Patienten erklärt. *Y. pseudotuberculosis* zeigt einen ähnlichen Infektionsverlauf wie *Y. enterocolitica,* wobei aber häufiger mesenteriale Lymphknoten, Leber und Milz betroffen sind und Enteritissymptomatik und die Ausscheidung über den Darm viel geringer sind als bei *Y. enterocolitica.*

Die enteritische Form der Yersinien-Infektionen, auch als Yersiniose bezeichnet, ist in unkomplizierten Fällen auf den Magen-Darm-Trakt begrenzt und durch mehrtägigen wässrigen Durchfall gekennzeichnet (sog. akute Yersinien-Gastroenteritis). Jedoch können Yersiniosen in Abhängigkeit vom Alter, dem Immunstatus, dem Histokompatibilitätstyp (HLA-B27) und dem Geschlecht der Patienten unterschiedliche Verlaufsformen und Symptome aufzeigen, wie z.B. Fieber, Übelkeit, kolikartige Unterbauchschmerzen (Pseudoappendizitis), Muskel- und Kopfschmerzen und Entzündungen im Rachenbereich (Pharyngitis), in den mesenteriale Lymphknoten (akute mesenteriale Lymphadenitis) und im Ileum und Kolon (Yersinia-Iletitis und -Colitis). In unkomplizierten Fällen klingt die Erkrankung nach wenigen Tagen bis spätestens nach zwei Wochen ab.

Bei Patienten mit Grunderkrankungen wie z. B. *Diabetes mellitus,* Leberzirrhose, Eisenspeicherkrankheiten (Dialyse-Patienten, Thalassämie) oder einer Immunsuppression kann es zu extraintestinalen Infektionen kommen (z. B. septische Formen). Nicht selten kommt es nach einer Bluttransfusion mit Yersinia-kontaminierten Blutkonserven zum septischen Schock mit hoher Letalität (ca.75%).

Neben den intestinalen und extraintestinalen Formen der Yersiniose, bei denen der Erreger meist isoliert werden kann, treten in bis zu 30% der Fälle noch Begleit- oder Folgeerkrankungen auf, die meist einen aseptischen Verlauf haben. Die häufigste Folgeerkrankung ist die HLA-B27-assoziierte Reaktive Arthritis [kurz: ReA] (etwa 60-80% der ReA -Fälle sind HLA-B27 positiv). Die ReA beginnt ca. 1-4 Wochen nach dem Auftreten der intestinalen Symptome (oft aber ohne vorangegangener Darmsymptomatik). Dabei sind am häufigsten die Gelenke der unteren Extremitäten betroffen. In den meisten Fällen klingt die Symptomatik nach ca. 3-12 Monaten ab, der Verlauf kann sich aber auch chronisch oder rezidivierend entwickeln. Weitere häufig auftretende Komplikationen nach einer Yersiniose sind Entzündungen der Schilddrüse (Thyroiditis), des Herzmuskels (Myokarditis) und der Nieren (Glomerulonephritis), rötliche Hauterscheinungen (Erythem) in den unteren Extremitäten (*Erythema nodosum*) und akute oder chronische Vergrößerungen der Lymphknoten (Lymphadenopathien) oder der Milz (Splenomegalien).

Bei Yersiniosen ohne weitere immunologische Komplikationen, wie z. B. reaktive Arthritis, kommt es meist nach kurzer Zeit zum Abklingen der Symptome. Damit ist eine Behandlung einer Yersinien-Infektion mit Antibiotika in den meisten Fällen unnötig. Bei septischen Verläufen oder lang anhaltenden Symptomen ist eine differenzierte Behandlung mit Antibiotika notwendig. Wegen der häufig vorkommenden Betalaktamasen bei Yersinien werden Infektionen mit *Y. enterocolitica* oder *Y. pseudotuberculosis* erregerspezifisch mit Gyrasehemmern, Tetracyclin, Trimethoprim/Sulfmethoxazol u.a. behandelt.

Die Unterscheidung verwandter Yersinien-Arten (z. B. *Y. enterocolitica* und *Y. pseudotuberculosis*) erfolgt biochemisch. *Y. pseudotuberculosis* bildet dabei eine biochemisch einheitliche Gruppe, die sich in acht Serogruppen aufteilt, von denen in Europa die Serovare I, II und III von humanmedizinischer Bedeutung sind (Bottone, [1997] Clin. Microbiol. Rev., 10, 257-276). Dagegen ist *Y. enterocolitica* eine sowohl biochemisch wie auch serologisch äußerst heterogene Art, bei der pathogene und apathogene geographisch getrennte Untergruppen vorkommen. *Y. enterocolitica* wird in sechs Biovare(BV)/Biotypen mit unterschiedlichen biochemischen Leistungen (1A, 1B, 2, 3, 4. 5) und ca. 60 Serovare (anhand unterschiedlicher O- und H-Antigene) (Wauters et al., [1987] Contrib. Microbiol. Immunol., 9, 14-21) unterteilt. Während Biovar 1A Yersinien hauptsächlich in der Umwelt vorkommen, gehören die bekannten humanpathogenen *Y. enterocolitica* -Stämme zu den BV 1B (Serotypen O:8, O:4, O:13, O:18, O:20, O:21, hauptsächliches Vorkommen in den USA), BV 2 (Europa [O:9], USA, Japan [O:5, 27]), BV 3 (O:9 und O:5, 27) und BV 4 (Europa und USA [O:3]). (Bucher et al., [2008] Foodborne Pathog. Dis., 5, 273-280). Ca. 80-90% der gemeldeten Yersinien-Gastroenteritiden in Deutschland werden durch *Y. enterocolitica* -Serotyp O:3 verursacht (RKI).

Die Virulenz der enteropathogenen Yersinien (*Y. enterocolitica* Biovare 1B, 2, 3, 4 und 5, *Y. pseudotuberculosis*) und des Pesterregers *Y. pestis* wird durch ein hoch konserviertes 65- - 70-kp großes Virulenzplasmid pYV (in *Y. pestis* pCDI) hervorgerufen. Dessen Virulenzfaktoren ermöglichen pathogenen Yersinien im lymphoidalen Gewebe des Wirtes zu überleben. Der *Y. enterocolitica* -Biovar 1A trägt kein pYV-Plasmid und wird daher als apathogen betrachtet.

Das pYV-Plasmid kodiert für das Membranprotein YadA (*Yersinia* Adhäsin) sowie eine Reihe von sog. *Yersinia* outer proteins (Yops) und den zugehörigen Sekretionsapparat (T3SS), der als Ysc (Yop secretion) bezeichnet wird. Als Substrat des T3SS werden die Yops als Anti-Wirts-Effektoren in eukaryontische Zellen (z. B. in Makrophagen) injiziert. Der Proteinkomplex des T3SS besteht aus 27 Proteinen mit bekannten oder unbekannten Funktionen, wie z. B. einer regulatorischen Funktion (YopQ, YopN), Translokationsfunktion (YopB, YopD) und Effektorfunktion (YopH, YopE, YopT, YopP, YopO). Das Adhäsin YadA wird ebenfalls auf dem Virulenzplasmid kodiert. Es vermittelt die Adhäsion der Erreger an Wirtszellen und ermöglicht die Yops in die Zielzellen zu injizieren. Das sogenannte V-Antigen (LcrV) ist ebenfalls auf dem pYV-Plasmid kodiert. Dieses Antigen bildet die Spitze des Ysc-Injektisoms, reguliert die Yop-Sekretion und Modulation des Immunsystems des Wirts.

Die Virulenz der Yersinien wird abgesehen von den plasmidkodierten Virulenzfaktoren auch durch chromosomal kodierte Faktoren beeinflusst. Die Adhäsion der Yersinien an Epithelzellen des Darms erfordert unter anderem das Vorhandensein des Invasins Inv und des Adhäsins Ail (sog. "attachment invasion locus" der Y. *enterocolitica*), welche von allen enteropathogenen Yersinien exprimiert werden können.

Weitere bekannte chromosomal kodierte Pathogenitätsfaktoren sind z. B. das sezernierte und hitzestabile Enterotoxin Yst, das sog. "Mucoid *Yersinia* factor" MyfA von *Y. enterocolitica,* bzw. das homologe pH 6-Antigen PsaA von *Y. pseudotuberculosis* und *Y. pestis,* das auf der sogenannten Hochpathogenitätsinsel (HPI) kodierte Yersiniabactin-Siderophorsystem (FyuA-Rezeptor mit Irp1-9), der Ysa Type-III-Sekretionsapparat, das für alle gram-negativen Bakterien charakteristische Lipopolysaccharid LPS, das Enzym Urease und der auf Insekten wirkende Toxinkomplex TC (*tcbA, tcaC, tccC*).

Das MyF-Fibrillensystem besteht aus den drei Untereinheiten MyfA, MyfB und MyfC. Die Fibrille wird aus MyfA-Untereinheiten aufgebaut, während die MyfB- und MyfC den Transport- und Aufbauapparat bilden. Das ca. 17-kD-große MyfA-Oberflächenantigen wird *in vitro* während der frühen stationären Wachstumsphase exprimiert, und die volle Expression erfolgt nur bei 37°C in saurem Milieu (pH 6). Das *myfA*-Gen ist in humanpathogenen *Y. enterocolitca* -Stämmen (z. B. O:3, O:4, O:8; O:9) und in ca. 16% der nicht-humanpathogenen *Y. enterocolitica* BV 1A -Stämmen vorhanden und zeigt einen signifikanten Zusammenhang mit der Virulenz. Leiva und Mitarbeiter zeigten in Koagglutination-Experimenten mit Seren aus Kaninchen, die mit lebenden *Y. enterocolitica*- oder *Y. pseudotuberculosis*-Stämmen intravenös immunisiert wurden, dass die erhaltenen Antiseren gegen MyfA (*Y. enterocolitica*) bzw. PsaA (*Y. pseudotuberculosis*) eine bakteriologische Differenzierung zwischen *Y.enterocolitica* (MyfA)- und *Y.pseudotuberculosis* (PsaA)-Stämmen erlauben (Leiva et al., [1995] Contrib. Microbiol. Immunol., 13, 158-164). Diese Immunisierungsexperimente zeigen auch, dass MyfA bzw. PsaA unter Laborbedingungen, d.h. unter kontrollierten Nährstoffbedingungen (Wachstumsmedium) sowie Temperatur- und pH-Bedingungen, produziert werden und die Produktion der Serumantikörper in intravenös immunisierten Kaninchen induzieren. Eine serodiagnostische Anwendung beim Menschen wird jedoch nicht in Betracht gezogen. Insbesondere, da die natürliche Funktion der Antigene nicht bekannt ist, kann auch nicht ohne weiteres erwartet werden, dass das MyfA- bzw. PsaA-Antigen während einer natürlichen Infektion im menschlichen Darmtrakt von pathogenen Yersinien exprimiert werden. Zusätzlich ist zu beachten, dass tierexperimentelle Daten selten auf die diagnostische Situation am Menschen übertragbar sind, weil bekanntermaßen die Produktion von Antikörpern in Tieren sich von der im Menschen unterscheiden kann.

Der Psa-Antigenkomplex (pH6-Antigen) von enteropathogenen *Y. pseudotuberculosis* wurde ursprünglich im Pesterreger *Y. pestis* als fimbrilläre Struktur mit einem Durchmesser von 3-5 nm charakterisiert. Die PsaA-Untereinheiten werden auf der Bakterienoberfläche durch den Translokations- und Aufbauapparat, der aus PsaB und PsaC besteht, angeordnet. Die ca. 17-kD-große Antigendomäne PsaA zeigt ca. 44-47%-ige Aminosäuresequenzhomologie mit den *Y. enterocolitica*-homologen MyfA. Wie beim MyfA wird die Produktion von PsaA in Yersinien durch die Temperatur (37°C) und den leicht sauren pH-Wert (pH 6) unter Laborbedingungen induziert. Die PsaA-Deletionmutantenstämme von *Y. pestis* zeigen eine signifikant reduzierte Virulenz *in vitro* und *in vivo.*

Die Beobachtungen, dass MyfA- und PsaA-Antigene unter Laborbedingungen nur bei 37°C und in saurem pH exprimiert werden und, dass eine Immunantwort in Labormäusen und -kaninchen nach einer Infektion mit vorkultivierten (in vitro) Yersinienstämmen hervorgerufen werden kann, führt zur Vermutung, dass diese Proteine eine Funktion während einer Infektion haben könnten bzw. in dem sauren Milieu des Darmtraktes exprimiert werden könnten. Jedoch sind die genauen Funktionen von Myf und Psa bei einer Yersinieninfektion, und somit die Bedeutung dieser Antigene für die Yersinien-Pathogenese, weitergehend unbekannt.

Die klassische Diagnose der akuten Yersiniose-Erkrankungen stützt sich primär auf den Erregernachweis im Stuhl, z. B. mittels Kälteanreicherung, selektivem Nährboden (z. B. sog. cefsulodin-Irgasan™-novobiocin Agarnährboden [CIN-Agar]), biochemischen Eigenschaften (z. B. sog. API E20-Test; bioMerieux, Paris, Frankreich) und den Nachweis von Erreger-spezifischer Nukleinsäuren (DNA) mittels Polymerase-Ketten-Reaktion (PCR, engl. "polymerase chain reaction"). Der Keimnachweis im Stuhl kann über eine Zeitdauer von 2 bis 12 Wochen möglich sein. Nach Abklingen der Durchfallsymptomatik sind Yersinien im Stuhl typischerweise nicht mehr nachzuweisen.

Die Serologie, d. h. der Nachweis der individuellen Serumantikörperantwort gegen Yersinien-spezifische O- und H-Antigene (sog. Widal- und passive Hämagglutinations-Tests), virulenzassoziierte Proteine (z. B. mittels Enzyme-linked Immunosorbent Assay [ELISA] - und Immunoblot -Teste) oder Bakterienultrasonikat (mittels Komplementbindungsreaktion [KBR]), ist nach geltenden Standards zur ergänzenden Diagnostik der akuten Infektion geeignet.

Jedoch ist die Serodiagnostik für die Aufklärung von Folgeerkrankungen, wie z. B. der reaktiven Arthritis, essentiell, weil der direkte Erregernachweis meistens nach Abklingen der akuten Infektion nicht möglich ist. Dir vorliegende Erfindung betrifft Vorrichtungen zur Diagnostik mit deren Hilfe zwischen einer Infektion mit *Yersinia enterocolitica* von einer Infektion mit *Yersinia pseudotuberculosis* serologisch unterschieden werden kann.

Bei einer akuten Yersinien-Infektion sind typischerweise die Yersinia-spezifische Immunoglobulin (Ig) -Klassen IgM-, IgA- und meist auch IgG nachweisbar. Im Verlauf der Infektion schwächt sich die spezifische IgM- und IgA -Antwort innerhalb von 3-6 Monaten ab (Persistenz von IgM: ca. 1-3 Monate und IgA: ca. 2-4 Monate), während Yersinien-spezifische IgG-Antikörper mehrere Jahre, eventuell auch lebenslang persistieren können (bei 80% der Patienten nach einer Yersinien-Infektion). Bei der chronischen Yersinien-Infektion und der Yersinien-induzierten reaktiven Arthritis können über Jahre, neben Yersinia-spezifischen Antikörper der IgG-Klasse auch persistierende IgA-Antikörper nachgewiesen werden. Die Yersinien-spezifischen Antikörper der IgM-Klasse sind bei Folgeerkrankungen meistens nicht mehr nachweisbar.

Die herkömmlichen serologischen Nachweismethoden, wie die gegen Ganzzellsysate gerichtete Widal- und Komplementbindungsreaktion, besitzen aufgrund der Kreuzreaktivität mit einer Vielzahl von humanen Krankheitserregern (wie z. B. *Bartonella henselae, Borrelia burgdorferi, Chlamydia pneumoniae, Rickettsia rickettsii, Escherichia coli, Brucella* spp., *Salmonella* spp.) nur eine geringe diagnostische Sensitivität und Spezifität. Daher werden heute Enzymimmunoassays (ELISA) und Immunoblots zum Nachweis von IgG-, IgA- und IgM-Antikörpern gegen rekombinant hergestellte virulenzassoziierte Yersinien-spezifische Antigene (z. B. Yops und V-AG) bevorzugt.

Die gegenwärtige Yersinien-Serodiagnostik basiert hauptsächlich auf der Nachweisreaktion der IgG- und IgA- (bedingt auch IgM-) Antwort gegen Virulenzplasmid pYV sezernierte Yop-Proteine, wie z. B. YopD, YopH, YopM, YopE, V-AG und YopN. Die Spezifität und Serisitivität dieser Yop-Antigene ist jedoch verbesserungswürdig und wird erfindungsgemäß durch zusätzliche Yersinien-spezifische Antigene ergänzt.

Zusätzlich bedingt durch die Ähnlichkeit der Ätiologie und des Infektionsvorgangs, einen subklinischen Infektionsverlauf bzw. eine unspezifische Symptomatik sowie eine hohe Durchseuchungsrate, ist die Unterscheidung zwischen *Y. enterocolitica* und *Y. pseudotuberculosis* -Infektionen bzw. durch diese Erreger hergerufene Folgeerkrankungen (wie z. B. ReA bzw. postenteritische Arthritiden, Myokarditis, Glomerulonephritis, Lymphadenopathien, Splenomegalien, *Erythema nodosum*) mit den herkömmlichen diagnostischen Testverfahren schwierig. Ein spezifisches serologisches Nachweisverfahren (d. h. insbesondere Differenzierung zwischen den häufigsten humanpathogenen Yersinien-Arten *Y. enterocolitica* und *Y. pseudotuberculosis*) ist jedoch erforderlich, um eine frühzeitige und effektive Behandlung der Yersiniosen bzw. eine Vorbeugung der Folgeerkrankungen gewährleisten zu können (d. h. adäquate Antibiotikatherapie). Hier können die unterschiedlichen Proteine von *Y. enterocolitica* (MyfA) und *Y. pseudotuberculosis* (PsaA) einen Beitrag leisten.

Heesemann et al. (Microbial Pathogenesis [1988], S. 437-447) beschreiben die Immunantwort von oral infizierten Kaninchen auf virulente (pYV-Plasmid) und avirulente (kein pYV-Plasmid) Serotyp 0:3-Stämme von *Yersinia enterocolitica,* die über Nacht in neutralem Wachstumsmedium (BHI) vorkultiviert wurden. Es ist unwahrscheinlich, dass das MyfA-Antigen unter diesen Bedingungen exprimiert wurde aufgrund neutralen pHs und der Wachstumsphase. Zusätzlich ist das Vorhandensein des PsaA-Antigens ausgeschlossen (Die Autoren setzten nur *Y.enterocolitica*-Stämme ein.). Die Antigene wurden mit SDS-Elektrophorese aufgetrennt und im Western Blot weiter untersucht. Eine weitergehende Reinigung erfolgte nicht. Weiterhin stellten die Autoren fest, dass dieses Verfahren aufgrund hoher Kreuzreaktivität mit darmpathogenen *Escherichia coli* und *Salmonella-*Stämmen als solches für diagnostische Zwecke eher ungeeignet ist.

Tomaso et al. (European Journal of Epidemiology [2006], 21: 77-81) und Stolck-Engelaar et al. (Scand.J.Infect.Dis. [1996], S. 571-575) beschreiben die Seroprevalenz von Anti-Yersina-Antikörpern in gesunden Österreichern bzw. an Yersinose erkrankten Niederländern. Die Bestimmung erfolgt mittels eines kommerziellen Western Blot-Tests mit den Antigenen Yop M, Yop H, V-Ag, Yop D und Yop E. Die Antigene MyfA oder PsaA werden nicht verwendet.

Das einzige bis jetzt vorgeschlagene Verfahren basierend auf den MyfA- und PsaA-Antigenen wurde von Leiva et al., 1995, beschrieben. Dabei handelte es sich aber um eine bakteriologische Nachweismethode zur Identifizierung von pathogenen YersinienStämmen nach Anzucht der Zellen auf einem Agarnährboden.

Gegenstand der vorliegenden Erfindung ist eine Vorrichtung gemäß den Ansprüchen zur serologischen Unterscheidung einer Infektion mit *Yersinia enterocolitica* von einer Infektion mit *Yersinia pseudotuberculosis.* Eine "serologische Unterscheidung" im Sinne der vorliegenden Anmeldung bedeutet, dass anhand einer aus Blut (Serum, Plasma) gewonnenen Probe mit Hilfe eines immunologischen Tests bestimmt werden kann, ob es sich um eine Infektion handelt, die durch einen Stamm von *Yersinia enterocolitica* oder *Yersinia pseudotuberculosis* hervorgerufen wurde. Eine derartige Vorrichtung enthält wenigstens ein Antigen ausgewählt aus einer Gruppe von Antigenen, die den äußeren Oberflächenproteinen oder sezernierten Proteinen von Yersinia zugerechnet werden können. In der Vorrichtung muss wenigstens eines dieser Antigene eingesetzt werden, wobei es nicht zwangsläufig erforderlich ist, dass das komplette Protein eingesetzt wird, sondern es kann durchaus genügen, Proteinfragmente einzusetzen, die ein diagnostisch relevantes Epitop aufweisen.

Erfindungsgemäß werden die Antigene in im wesentlichen reiner Form eingesetzt, dies wird vorzugsweise dadurch erreicht, dass die Antigene rekombinant hergestellt und nicht aus Zelllysat isoliert werden.

Ausreichend ist ein Fragment eines der nachfolgend aufgezählten Antigene, das mindestens acht aufeinanderfolgende Aminosäuren, bevorzugt mindestens 12, noch bevorzugter mindestens 20, weiter bevorzugt wenigstens 30 aufeinanderfolgende Aminosäuren und ganz besonders bevorzugt wenigstens 50 aufeinanderfolgende Aminosäuren aufweist. In einer bevorzugten Ausführungsform weisen die Peptide 10 bis 30 aufeinanderfolgende Aminosäuren auf. Jedes Peptid/Fragment weist wenigstens ein diagnostisch relevantes Epitop auf.

Bei der Auswahl der Fragmente wird ein solcher Bereich ausgewählt, der wenigstens ein diagnostisch relevantes Epitop enthält. Die Lokalisierung der Epitop-Bereiche kann durch dem Fachmann bekannte Standardmethoden erfolgen. Es ist möglich, die Hydrophilizität/Hydrophobizität des Proteins mit geeigneten Computerprogrammen zu bestimmen. Hydrophile Bereiche sind in der Regel dazu prädestiniert geeignete Epitope zu tragen, da die hydrophilen Bereiche im gefalteten Protein an der Oberfläche zu liegen kommen. Hydrophobe Bereiche werden eher im Inneren des gefalteten Proteins lokalisiert sein und sind daher an diagnostisch relevanten Epitopen eher nicht beteiligt. Bei den Epitopen handelt es sich bevorzugt um lineare Epitope, Konformationsepitope können jedoch auch vorteilhafterweise eingesetzt werden.

Wenn geeignete Bereiche identifiziert wurden, können diese entweder durch chemische Synthese synthetisiert werden oder rekombinant hergestellt werden. Diese Proteine oder Peptide können dann mit geeigneten Blut-, Serum- oder Plasmaproben, deren Ätiologie mit anderen, medizinischen Parametern bestimmte wurde, umgesetzt werden. Auf diese Art und Weise kann der Fachmann geeignete Epitope lokalisieren.

Die erfindungsgemäßen Vorrichtungen enthalten also wenigstens ein Antigen bzw. ein Fragment eines dieser Antigene ausgewählt aus der Gruppe bestehend aus Antigenen, die nachfolgend aufgezählt sind. Es handelt sich hierbei um folgende Antigene:
**YopD (Seq ID No. 1)**
**YopH (Seq ID No. 2)**
**YopN (Seq ID No. 3)**
**YopE (Seq ID No. 4)**
**YopM (Seq ID No. 5)**
**V- AG (auch LCRV genannt) (Seq ID No. 6)**

Zusätzlich zu dem Antigen aus der ersten Gruppe von Antigenen weist die erfindungsgemäße Vorrichtung weiterhin wenigstens eines von zwei weiteren Proteinen, nämlich entweder das Protein MyfA und/oder das Protein PsaA oder Fragmente eines dieser beiden Proteine auf. Auch hier weisen die Fragmente eine Mindestgröße von wenigstens 8 aufeinanderfolgenden Aminosäuren, bevorzugt wenigstens 12 aufeinanderfolgenden Aminosäuren, noch bevorzugter wenigstens 20, besonders bevorzugt wenigstens 30 und ganz besonders bevorzugt wenigstens 50 aufeinanderfolgenden Aminosäuren eines der Proteine MyfA und/oder PsaA auf.

Es ist bevorzugt, dass die Vorrichtung die beiden vollständigen Proteine MyfA und PsaA oder Fragmente davon zusammen aufweist, wobei die einzelnen Antigene räumlich voneinander getrennt sind. Die Aminosäuresequenzen der beiden Proteine MyfA und PsaA (ohne Leader-Sequenz) sind nachfolgend wiedergegeben.
**MyfA** (132-AA) (Seq ID No. 7)
**PsaA** (134-AA) (Seq ID No. 8)

Die Proteine MyfA bzw. PsaA werden durch die nachfolgend wiedergegebenen Nucleotidsequenzen *myfA* bzw. *psaA* codiert und können unter Verwendung geeigneter Vektoren und Wirtszellen rekombinant hergestellt werden.
***myfA*** (Seq ID No. 9)
***psaA*** (Seq ID No. 10)

Die einzelnen Antigene werden erfindungsgemäß räumlich getrennt voneinander in der Testvorrichtung bzw. dem Testkit angeordnet. Bei Western Blots beispielsweise können die Antigene in Bandenform auf das Trägermaterial aufgebracht werden, wobei die einzelnen Antigene jeweils in einer bestimmten, wohldefinierten Bande vorhanden sind. Diese räumliche Trennung der einzelnen Antigene kann ebenso erreicht werden, wenn die Antigene in einem Line-Assay räumlich getrennt voneinander aufgebracht werden. Es ist aber auch möglich, die einzelnen Antigene auf einer Mikrotiterplatte so aufzubringen, dass in jeder Vertiefung nur ein Antigen vorhanden ist. In einer alternativen Ausführungsform wird jeweils ein Antigen auf einem Typ von Trägern (beispielsweise Kugeln) aufgebracht, so dass auf jeden Träger nur ein Antigen gebunden ist. Alternativ hierzu können die Antigene auf Testplatten (Microchips) aufgebracht werden, wobei auf derartigen Chips die einzelnen Antigene an bestimmten Punkten fixiert sind.

Erfindungsgemäß wird zunächst durch Reaktion mit wenigstens einem, oder auch mehreren Antigenen ausgewählt aus der Gruppe Yop D, Yop H, Yop M, Yop E, V-AG und/oder Yop N bestimmt, ob es sich um eine Yersinien-Infektion handelt. Wenn dieser Test positiv ausgefallen ist, wird mit Hilfe des Antigens MyfA und/oder PsaA bestimmt, ob es sich bei dem Infektionsverursacher um *Yersinia enterocolitica bzw. Y.pseudotuberculosis* handelt. Die einzelnen Nachweisschritte können entweder gleichzeitig oder nacheinander durchgeführt werden.

Bei einer Vorrichtung zum serologischen Nachweis einer Infektion von Yersinia-Species handelt es sich erfindungsgemäß um einen diagnostischen Kit. Darunter versteht man eine Vorrichtung, die von diagnostisch tätigen Labors zur serologischen Diagnose eingesetzt werden. In bevorzugter Ausführungsform werden die Antigene räumlich voneinander getrennt auf eine Trägermatrix, wie z.B. Vertiefungen einer Mikrotiterplatte, Kugeln, Nitrocellulose oder Nylon, gebunden. Die in eine Patientenprobe (z.B. Blut, Serum, Plasma, Saliva) vorhandenen Antikörper (vor allem der Klassen IgG, IgM und IgA) reagieren mit den gebundenen Antigenen und werden dabei immobilsiert. In bevorzugter Ausführungsform handelt es sich hierbei um diagnostische Kits, wobei ELISA-Tests eine bevorzugte Ausführungsform darstellen. Bei den ELISA-Testkits wird üblicherweise das Antigen an die Vertiefungen einer Mikrotiterplatte gebunden. Die in den Proben vorhandenen spezifischen Antikörper können mit den Antigenen reagieren. Die Antikörper aus dem Serum oder Plasma, die an die in den Vertiefungen befindlichen Antigene spezifisch gebunden haben, werden in der Regel mit Anti-Antikörpern, die eine Markierung, vorzugsweise eine Enzym-Markierung, tragen, nachgewiesen.

Eine andere Ausführungsform der erfindungsgemäßen Vorrichtung sind sogenannte Line-Tests. Hierbei werden mehrere Antigene nach einem vorbestimmten Muster auf die Teststreifen aufgebracht. Die zu untersuchenden Blutproben (Seren oder Plasmen) werden mit den Teststreifen umgesetzt und Antigen-Antikörper Reaktionen werden durch Enzym-markierten Antikörper und anschließende Färbereaktion nachgewiesen. Aus dem spezifischen Muster der Reaktivitäten bzw. Farbsignale kann auf die Infektion bzw. Infektionsverursacher geschlossen werden.

Bei der erfindungsgemäßen Vorrichtung kann es sich auch um einen Immunoblot bzw. Western Blot handeln. Hierbei werden die diagnostisch relevanten Proteine zunächst der Größe nach beispielsweise durch Diffusion, Kapillarwirkung oder Elektrophorese aufgetrennt und auf ein Trägermaterial, beispielsweise eine Nylonmembran oder eine Nitrocellulosemembran übertragen und dort fixiert. Dieses Trägermaterial mit den daran gebundenen Proteinen bzw. Proteinfragmenten wird mit Blutproben (Serum oder Plasma) von Patienten umgesetzt.

Die immobilisierten spezifischen Antikörper können beispielsweise durch Reaktion mit einem Anti-Antikörper nachgewiesen. Es können bevorzugt verschiedene Anti-Antikörper eingesetzt werden, die entweder mit IgG, IgM oder IgA reagieren. Dadurch wird eine weitere Differenzierung der Immunantwort ermöglicht. Diese Anti-Antikörper tragen in der Regel eine Markierung. Hierbei kann es sich um ein Enzym handeln, das eine Farbreaktion katalysiert, es kann sich aber auch um fluoreszierende Reste oder radioaktive Reste handeln. Wichtig ist, dass mit den Anti-Antikörpern an Antigen gebundene Antikörper nachgewiesen werden können.

In einer anderen Ausführungsform handelt es sich bei der erfindungsgemäßen Vorrichtung um einen auf Kügelchen basierenden Test (sog. Bead-Based-Assay). Eine bekannte kommerzielle Anwendungsform von diesen sphärischen Mikroarrays stellt die Luminex-XMAB-Technologie von Luminex Corporation (Austin, USA) dar. Bei diesem System werden Mikrokugeln (sog. Beads) eingesetzt und mit Hilfe der Flow-Cytometrie ausgewertet. Bei Fluoreszenz-markierten Ausführungsformen können die erfindungsgemäßen Antigene auf Kugeln fixiert werden und die Bindung der Antikörper an die Antigene wird durch geeignete Markierungen, z.B. Fluoreszenz sichtbar gemacht.

Eine weitere Ausführungsform der erfindungsgemäßen Vorrichtung sind Protein-Mikroarrays. Hierbei werden verschiedene Antigene auf engem Raum an eine Oberfläche fixiert. Da bekannt ist, welches Antigen an welcher Stelle vorhanden ist, kann nach Sichtbarmachung der Antigen-Antikörperreaktion beispielsweise mit Hilfe einer Farbreaktion oder Fluoreszenz-Markierung auch eine Aussage getroffen werden, welches Antigen mit den Antikörpern in dem Serum reagiert hat.

Üblicherweise werden die einzelnen Antigene so in der Vorrichtung fixiert, dass nach der Reaktion unterscheidbar festgestellt werden kann, mit welchem Antigen die in der Probe vorhandenen Antikörper reagiert haben. Bei einem ELISA-Test werden die einzelnen Antigene in verschiedene Kavitäten der Mikrotiterplatte eingebracht. Beim Line-Assay werden die Antigene auf verschiedene Streifen des Trägermaterials aufgesprüht und bei den sphärischen und planaren Mikroarrays werden die jeweiligen Antigene immer an definierter Stelle auf den Träger bzw. definierten Beads aufgebracht. Die erfindungsgemäßen Vorrichtungen dienen dem Nachweis von humanpathogenen *Yersinia*-Species bzw. Subspecies. Durch die Kombination von verschiedenen Antigenen ist es einerseits möglich, einen sensitiven und spezifischen Nachweis der Erreger zu führen und andererseits ist in bevorzugter Ausführungsform auch ein differentialdiagnositischer Nachweis möglich, wobei zwischen einer Infektion verursacht durch *Yersinia enterocolitica* unterscheiden werden kann von einer Infektion mit *Yersinia pseudotuberculosis.*

Ein anderer wesentlicher Aspekt der vorliegenden Erfindung ist auch, dass ein serologischer Nachweis bzw. eine serologische Differenzierung dann möglich ist, wenn die akute Yersinieninfektion bereits abgeklungen ist. Für die Therapie von derartigen Folgeerkrankungen ist es wesentlich, dass unterscheiden werden kann, durch welches Bakterium (*Yersinia enterocolitica* oder *Yersinia pseudotuberculosis*) die ursprüngliche Infektion verursacht wurde.

Die erfindungsgemäße Vorrichtung weist also wenigstens zwei verschiedene Antigene auf, wobei das eine Antigen ausgewählt ist aus der Gruppe bestehend aus den oben näher dargestellten Antigenen YopD, YopH, YopM, YopE, V-AG und YopN. Die andere Gruppe umfasst die Antigene MyfA und PsaA.

In besonders bevorzugter Ausführungsform beinhaltet die Vorrichtung das Antigen YopD sowie das Antigen YopH zusammen mit entweder MyfA oder PsaA.

In einer noch weiter bevorzugten Ausführungsform beinhaltet die erfindungsgemäße Vorrichtung die Antigene YopD, YopH, YopM und entweder MyfA oder PsaA, wobei aber der kombinierte Einsatz von MyfA und PsaA ganz besonders bevorzugt ist.

In besonders bevorzugter Ausführungsform weist also die erfindungsgemäße Vorrichtung die Proteine MyfA und/oder PsaA oder Fragmente hiervon, wie vorstehend definiert, zusammen auf mit dem folgenden Protein bzw. Proteinkombinationen, wobei diese Proteine auch in Form von Fragmenten, wie vorstehend definiert vorliegen können. Die erfindungsgemäße Vorrichtung weist wenigstens das Protein YopD auf, das bevorzugt zusammen mit YopH und noch bevorzugter zusammen mit YopM kombiniert mit MyfA und/oder PsaA eingesetzt wird.

Eine weiter bevorzugte Ausführungsform beinhaltet die Proteine oder Fragmente von YopD, YopH, YopM und YopE zusammen mit MyfA und/oder PsaA.

Eine andere bevorzugte Ausführungsform beinhaltet die Proteine und/oder Fragmente von YopD, YopH, YopM, YopE, V-AG zusammen mit MyfA und/oder PsaA.

Eine weitere bevorzugte Ausführungsform beinhaltet eine Kombination von Proteinen und/oder Fragmenten der folgenden Bezeichnungen: YopD, YopH, YopM, YopE, V-AG, YopN, sowie MyfA und/oder PsaA.

Die genannten Antigene können auch in Form von Fragmenten davon eingesetzt werden, wobei die Fragmente ein serologisch relevantes Epitop aufweisen. Wenn erfindungsgemäß Fragmente der Antigene eingesetzt werden, ist bei dem Antigen MyfA einerseits das N-terminale Fragment mit den Aminosäuren 30-41 und andererseits das C-terminale Fragment mit den Aminosäuren 148-159 besonders bevorzugt. In dem PsaA Protein sind die Fragmente besonders bevorzugt, die die Aminosäuren 27-37 (N-Terminus) sowie 144-155 (C-Terminus) beinhalten. In diesem Fall werden Fragmente eingesetzt, die diese Aminosäuresequenz beinhalten und die oben näher definierten Größen aufweisen.

Gegenstand der vorliegenden Erfindung ist also die Verwendung eines antigenen Proteins MyfA oder eines Fragments hiervon, wie oben näher definiert, entweder allein oder zusammen mit dem antigenen Protein PsaA oder einem Fragment hiervon, wie oben definiert, zum serologischen Nachweis einer durch *Yersinia* verursachten Infektion bzw. einer Infektion verursacht durch *Y.enterocolitica* oder *Y.pseudotuberculosis.* Diese Unterscheidung hat klinische Relevanz, weil eine Infektion, verursacht durch *Yersinia enterocolitica,* anders behandelt werden kann, als eine Infektion hervorgerufen durch *Yersinia pseudotuberculosis.*

Mit der erfindungsgemäßen Vorrichtung können Yersinien nachgewiesen werden, die eine erhebliche Bedeutung haben, da sie humanpathogen sind. Die Yersinien kommen in Nutztieren, wie z.B. Schweinen und Geflügel, vor und können bei einer Infektion eine Erkrankung am Menschen hervorrufen. Es kann daher eine erhebliche medizinische und epidemiologische Bedeutung haben, mit Hilfe eines einfachen und verhältnismäßig kostengünstigen Testkits nachzuweisen, wie die Infektionsketten verlaufen sind. Dadurch sind Rückschlüsse auf die Quellen der Krankheitserreger möglich und diese können identifiziert und gegebenenfalls unschädlich gemacht werden. Es ist also möglich, die Erreger von Yersiniosen nachzuweisen und zu unterscheiden.

Die Ergebnisse, die mit dem erfindungsgemäßen Nachweis erzielt werden können, sind überraschend, weil nicht ohne weiteres angenommen werden konnte, dass die Antigene MyfA und/oder PsaA auch in einem menschlichen Organismus unter realen Infektionsbedingungen in einer zur Antikörperbildung ausreichende Mengen exprimiert werden. Weiterhin war es nicht bekannt, dass spezifische Antikörper der IgG-, IgM- und IgA-Klasse gegen MyfA- und PsaA-Antigene im Menschen gebildet werden.

Aufgrund der relativ hohen Proteinhomologie (44 %) zwischen MyfA- und PsaA-Antigenen und der bereits beschriebenen Interspezieshomologie bzw. Interspezieskreuzreaktivität zwischen nicht pathogenen Yersinienstämmen und anderen Enterobakterien, wie z.B. *E.coli* und *Salmonella*-Spezies, konnte nicht erwartet werden, dass das MyfA bzw. das PsaA-Antigen zur Differenzierung des Infektionsverursachers verwendet werden kann. Der Nachweis bzw. die Differenzierung wird bevorzugt mit menschlichem Blut, insbesondere Serum oder Plasma durchgeführt. Die Bestimmung ist aber auch mit Liquor oder Saliva möglich.

In einer ganz besonders bevorzugten Ausführungsform wird die zu untersuchende Probe einerseits mit einem Antigen ausgewählt aus der Gruppe Yop D, Yop H, Yop M, Yop E, V-AG und Yop-N, ganz besonders bevorzugt mit Yop D, umgesetzt. Wenn hier keine positive Reaktion erfolgt und auch sonst kein spezieller weiterführender Verdacht besteht, kann die Diagnostik hiermit abgeschlossen werden.

Wenn die zu untersuchende Probe dagegen mit diesem Antigen, insbesondere Yop D, positiv reagiert, kann in einem weiteren diagnostischen Schritt die zu untersuchende Probe mit dem Antigen PsaA und/oder MyfA, bevorzugt mit beiden Antigenen umgesetzt werden. Weiterhin hilfreich ist eine Differenzierung der aufgefundenen Antikörper dahingehend, ob es sich um IgG- oder um IgA-Antikörper handelt. Wenn der IgM- oder IgA-Befund negativ, der IgG-Befund aber positiv ist, liegt vermutlich eine abgelaufene Infektion vor.

Wenn der IgM- oder IgA-Befund, bevorzugt IgA-Befund, positiv ist, kann eine akute Infektion oder eine Folgeerkrankung vorliegen. Die Infektion wird hervorgerufen durch *Yersinia enterocolitica,* wenn die IgG-, IgM- oder IgA-Reaktion, bevorzugt IgG-Befund, mit MyfA positiv ist. Wenn eine positive Reaktion mit PsaA vorliegt, handelt es sich um eine Infektion mit *Yersinia pseudotuberculosis.*

Die Figuren verdeutlichen bevorzugte Ausführungsformen der vorliegenden Erfindung und erläutern die in den Beispielen erhaltenen Ergebnisse.

Figur 1 zeigt die homologen Aminosäuresequenzbereiche der MyfA- und PsaA-Antigene. Die homologen Bereiche (Konsensus) sind schwarz markiert. Die N- und C-terminalen homologen Bereiche sind umrandet.

Figur 2 erläutert die *In silico*-Bestimmung der antigenen Domänen von MyfA und PsaA. Der Antigenitätsindex wurde anhand des Jameson-Wolf -Algorithmus und die Hydrophilizität des Antigens anhand des Kyte-Doolittle -Algorithmus berechnet. Die sog. Leader-Sequenz ist mit einem schwarzen Pfeil markiert.

Figur 3 zeigt eine schematische Darstellung der DNA-Sequenzen der vier verwendeten His-Tag-MyfA-Fusionsproteine. Die zur Amplifikation verwendeten Primersequenzen sind mit Pfeilen (I, II, III, IV) markiert. Die Leader-Sequenzen sind hellgrau und die mit PsaAhomologen Bereichen weiß dargestellt.

Figur 4 zeigt die chromatographische Trennung (SDS-Polyacrylamid-Gelelektrophorese) der MyfA-Teilfragmente *myfA* 1-441, *myfA* 121-441 und *myfA* 121-447 (Fig. 3) mit anschließender Coomassie-Blau-Färbung (links) und Immunoblot (*myfA, myfA* 1-441, *myfA* 121-441 und *myfA* 121-447; Fig. 3) mit Anti-*Y. enterocolitica* (O:3 bzw. O:9)- und Anti-MyfA -Seren aus Kaninchen (rechts).

Figur 5 zeigt die Überprüfung der serologischen Reaktivität des aufgereinigten MyfA-Gesamtproteins (MyfA) und der aufgereinigten MyfA -Teilfragmente MyfA 1-441, MyfA 121-441 und MyfA 121-447 (Fig. 1; Fig. 3) mittels Line-Assays. Die Teststreifen wurden mit vier verschiedenen Anti-*Y. enterocolitica* O:3, O:8 bzw. O:9 -Seren aus Kaninchen (1-4) vor (*) und nach der Infektion inkubiert.

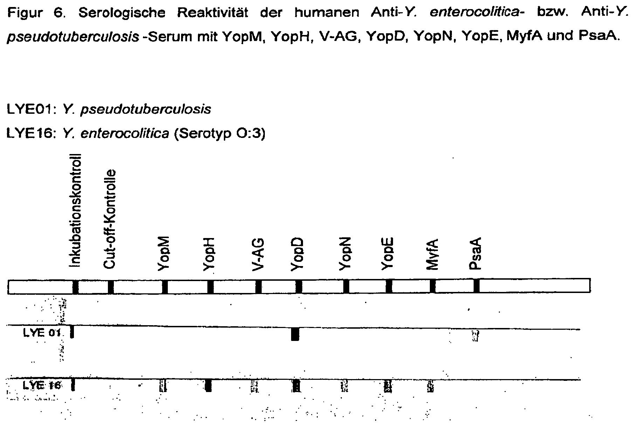

Figur 6 zeigt die Serologische Reaktivität der humanen Anti-*Y. enterocolitica-* bzw. Anti-*Y. pseudotuberculosis* -Serum mit YopM, YopH, V-AG, YopD, YopN, YopE, MyfA und PsaA im Line-Assay.

Figur 7 stellt die IgG-Antwort gegen Yop-, MyfA-MIK- und PsaA-MIK-Antigene unter den getesteten bayerischen Blutspenderseren (n=40) dar.

Figur 8 erläutert die IgG-Antwort gegen Yop-, MyfA-MIK- und PsaA-MIK-Antigene unter den getesteten Yersiniose-Patientenseren aus Finnland (n=18).

Figur 9 zeigt die IgG-Antwort gegen Yop-, MyfA-MIK- und PsaA-MIK-Antigene unter den getesteten Yersiniose-Patientenseren aus Deutschland (n=23). Unter dem Oberflächenprotein ist YopD für den Einsatz in diagnostischen Testvorrichtungen besonders geeignet.

### Beispiel 1

### In silico Bestimmung der immunogenen Domänen der MyfA- und PsaA-Antigene

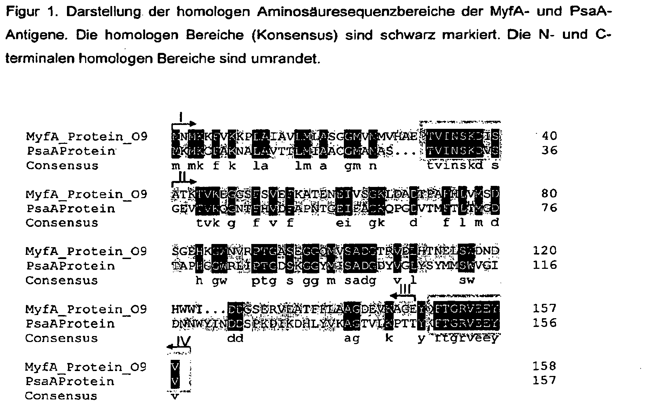

Die Bestimmung der homologen Antigenbereiche, die für die immunogene Reaktivität bzw. Kreuzreaktivität der MyfA- und PsaA- Antigene verantwortlich sein könnten, erfolgte *in silico* mittels direkten Aminosäuresequenzvergleichs (siehe Figur 1). Die homologen Bereiche befinden sich hauptsächlich im N- und C-Terminus des MyfA- (AA 32-40 und 150-159) und PsaA- (AA 29-38 und 149-158) Antigens.

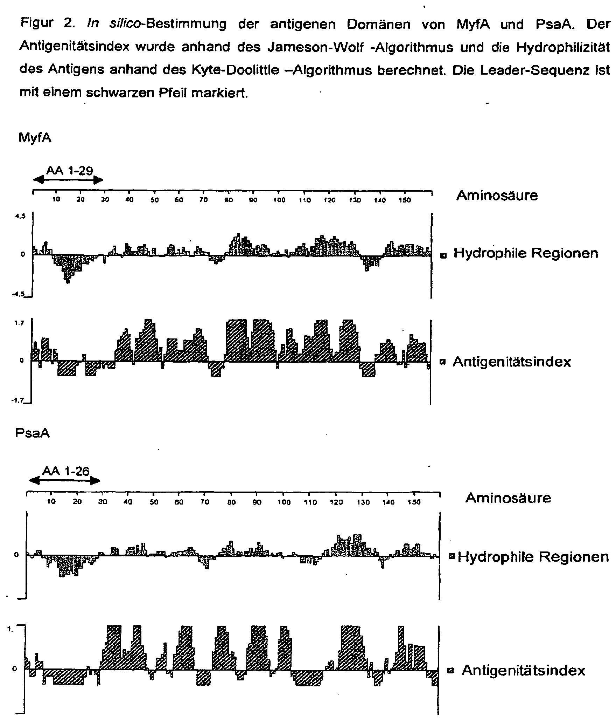

Der Antigenitätsindex und der hydrophile Charakter der Antigenregionen wurde *in silico* anhand der Algorithmen von Jameson und Wolf (Comput. Appl. Biosci. (1988) S. 181-186) bzw. von Kyte und Doolittle (Kyte and Doolittle, (1982) J. Mol. Biol., 157, S. 105-132) bestimmt (Fig. 2). Die acht > 5 AA immunogenen Domänen des PsaA-Antigens befinden sich voraussichtlich in AA 30-48, 52-56, 59-67, 74-83, 87-96, 122-133, 144-150 und 151-158. Die sieben putativen > 5 AA immunogenen Domänen des MyfA-Antigens wurden in AA 35-42, 43-54, 55-73, 79-100, 101-133, 139-148 und 152-159 lokalisiert. Diese Fragmente werden erfindungsgemäß bevorzugt in den diagnostischen Tests eingesetzt. Da jedoch der prädikative Kyte-Doolittle-Algorithmus nur bedingt aussagekräftig ist, wurden die immunogenen Epitope der neuen Antigene experimentell bestimmt (Beispiel 2).

### Vollständige Gesamtprotein-Sequenzen:

**MyfA** (159-AA) (Seq ID No. 11)
   AA 1-29: Leader-Sequenz
   AA 30-41: **N-terminales Fragment** (Beispiel 2)
   AA 148-159: **C-terminales Fragment** (Beispiel 2)
**PsaA** (158-AA) (Seq ID No. 12)
   AA 1-26: Leader-Sequenz

### Beispiel 2

### Experimentelle Bestimmung der immunogenen Domänen der MyfA-Antigene

### Herstellung der MyfA-Teilfragmente

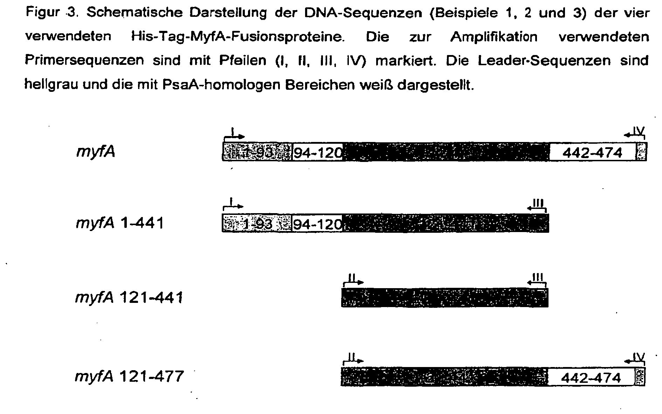

Ausgehend vom vollständigen Leserahmen des MyfA-Antigens (Beispiel 1) wurden vier Fragmente hergestellt. Dabei wurden sowohl der gesamte Leserahmen einschließlich des Leader-Peptids als auch N-, C-, und N- und C-terminal verkürzte Teilfragmente dargestellt (Fig. 1; Fig. 3).

Die spezifische Amplifikation der *myfA*-Teilfragmente erfolgte mittels PCR mit chromosomaler DNA aus *Y. enterocolitica* Serotyp O:3/4 (*Y. enterocolitica* subsp. *palearctica* Stamm Y-11; DMSZ 13030) als Template.

Die resultierenden Amplifikate wurden mittels Restriktionsendonukleasen enzymatisch gespalten und in einen geeigneten Vektor, pET21-b, der mit denselben Restriktionsendonukleasen wie die Amplifikate geschnitten wurde, ligiert. Nach der Transformation des Ligationsansatzes in einen geeigneten *E. coli*-Stamm, z. B. BL21 pLys, wurden einzelne Klone auf das Vorhandensein von *myfA* und *psaA* mittels Agarose-Gelelektrophorese enzymatisch gespaltener Plasmid-DNA getestet und anschließend sequenziert. Des Weiteren wurde die Expression der Teilfragmente durch Analyse der Expressionsprodukte mittels SDS-Polyacrylamid-Gelelektrophorese mit anschließender Coomassie-Blau-Färbung bzw. anschließendem Transfer auf Nitrozellulose gefolgt von einer immunologischen Detektion identifiziert und charakterisiert. Die Reinigung der Fusionsproteine erfolgte mittels Ni-NTA-Säulenchromatographie gemäß Herstellerangaben (Firma Qiagen).

Neben dem kompletten Leserahmen von MyfA (MyfA; Fig. 3) wurden drei Teilfragmente des Antigens als His-Tag-Fusionsproteine in Vektor pET21-b kloniert. Das erste Fragment (MyfA 1-441) wurde ohne den zu PsaA homologen C-terminalen Bereich (Beispiel 1; Fig. 1) kloniert und umfasst die AA 1-147 (Nukleotid [NT] 1-441). Das zweite Fragment (MyfA 121-441) kodiert für die AA 40-147 (NT 121-441) und enthält somit nicht die zu PsaA homologen N- und C-terminalen Bereiche (Beispiel 1; Fig. 1). Das dritte Fragment (MyfA 121-477) kodiert für die AA 40-159 (NT 121-477) und enthält nicht die N-terminale Sequenz von MyfA (Beispiel 1; Fig. 3; Fig. 1).

Nachfolgende Oligonukleotid-Primer wurden zur Darstellung der Sequenzen verwendet.
Primer 1 (I):
   myfA-f: GTA ATT CCA TAT GAA TAT GAA AAA ATT TGT (Seq ID No. 13)
Primer 2 (II):
   myfA_121f: GTA ATC CCA TAT GGC AAC AAA AAC TGT (Seq ID No. 14)
Primer 3 (III):
   myfA_441rev: TTA CTC GAG TTC ACC TGC TTT AAC (Seq ID No. 15)
Primer 4 (IV):
   myfA_rev: ATC TAC TCG AGC TCG ACA TAT TCC TCA A (Seq ID No 16)

Dabei wurden die folgenden Primerkombinationen verwendet:
*myfA*: Primer 1 und Primer 4
*myfA* 1-441: Primer 1 und Primer 3
*myfA* 121-441: Primer 2 und Primer 3
*myfA* 121-477: Primer 2 und Primer 4

### Beispiel 3

### Überprüfung der Antigenität

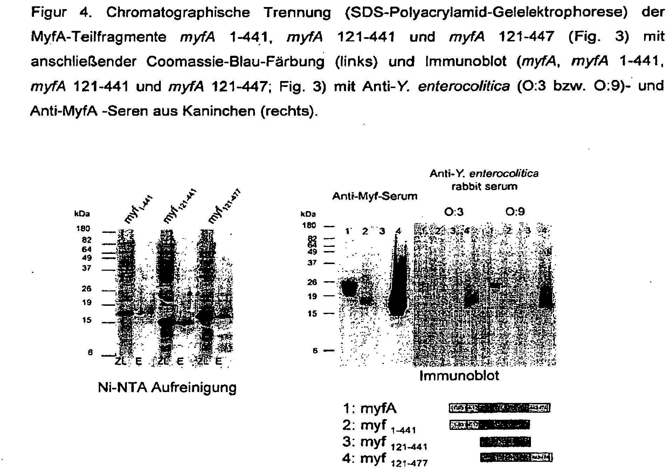

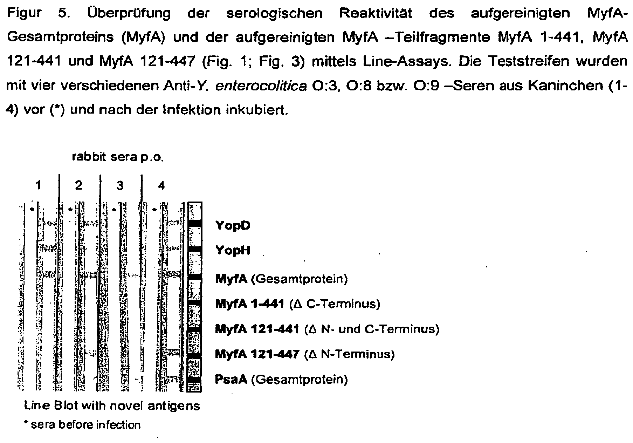

Die immunologische Reaktivität/Antigenität der MyfA-Fusionsproteine (Beispiel 2) wurde mittels Immunoblot (Fig. 4) und Line-Assay getestet (Fig. 5)

In Fig. 4 links sind jeweils Zellysate (ZL) von MyfA-Gesamtprotein (MyfA, inklusiver Leader-Sequenz; Beispiel 1; Fig. 1; Beispiel 2; Fig. 3) beziehungsweise eines der drei MyfA-Teilfragmente (MyfA 1-441, MyfA 121-441 und MyfA 121-447) exprimierenden *E. coli* BL21 pLys -Zellen sowie die mittels Ni-NTA-Säulenchromatographie aufgereinigten His-getaggten rekombinanten MyfA-Proteine (MyfA 1-441, MyfA 121-441 und MyfA 121-447) dargestellt.

Die rechte Darstellung in Fig. 4 zeigt einen Immunoblot mit zwei Anti-*Y. enterocolitica* O:3 bzw. O:9 - Seren und einem Anti-MyfA-Serum aus Kaninchen. Das am N- und C-Terminus verkürzte MyfA -Protein (MyfA 121-441; Beispiel 1; Fig. 1; Beispiel 2; Fig. 3) zeigt keine Reaktion mit den verwendeten Seren. Das am C-Terminus verkürzte Protein MyfA 1-441 (Fig. 1; Fig. 2) zeigt eine schwächere Reaktion als das am N-Terminus verkürzte Protein MyfA 121-447 (Fig. 1; Fig. 2). Somit scheint der C-Terminus einen besonders wichtigen immunogenen Bereich zu beinhalten, aber auch der N-Terminus ist diagnostisch bedeutend.

Durch Nachweis des His-tags mit einem Nickel-NTA-Konjugat aus Kaninchen konnten die Proteine nachgewiesen werden (sehr schwache Reaktion, keine Doppelbanden). Der Immunoblot mit einem Anti-Y. *pseudotuberculosis* -Serum aus Kaninchen zeigte keine Reaktion.

Die Überprüfung der immunologischen Reaktivität/Antigenität des aufgereinigten MyfA-Gesamtproteins (MyfA) und der aufgereinigten MyfA -Teilfragmente MyfA 1-441, MyfA 121-441 und MyfA 121-447 (Beispiel 1, Fig. 1; Beispiel 2; Fig. 3) erfolgte mittels Line-Assays (Fig. 5). Die Teststreifen wurden mit vier verschiedenen Anti-*Y. enterocolitica-*Seren aus Kaninchen (Serum 1 und 4: Serotyp O:3; Serum 2: Serum O:9, Serum 3: O:8) vor (*) und nach der experimentellen peroralen Infektion mit vorkultivierten Yersinien inkubiert. Als Immunisierungs- bzw. Assay-Kontrolle wurden rekombinant hergestellte YopD- und YopH-Antigene in den Assay eingebaut. Zusätzlich, um die Kreuzreaktivität der MyfA- und PsaA-Antigene evaluieren zu können, wurde auch rekombinant hergestelltes PsaA-Antigen in den Assay eingefügt (Fig. 5). Die Reaktivität der Antigene wurde außerdem mit einem Anti-Y. *pseudotuberculosis* -Serum aus Kaninchen überprüft.

Die vor Infektion abgenommenen Blutproben zeigten keine Reaktivität mit den Antigenen des Assays (d. h. YopD, YopH, MyfA, MyfA 1-441, MyfA 121-441, MyfA 121-447, PsaA). Nach der experimentellen Infektion reagierten alle vier Proben mit den Yersinien-spezifischen Antigenen YopD und YopH (Fig. 5). Wie schon beim Immunoblot dargestellt (Fig. 4), zeigten das C- (Δ C-Terminus) bzw. das N- und C- (Δ N- und C-Terminus) terminal verkürzte MyfA-Protein deutlich verminderte und das N-terminal verkürzte Protein (Δ N-Terminus) verminderte Reaktivität im Vergleich zum MyfA-Gesamtprotein (Fig. 5). Interessanterweise reagierten die Seren Nr. 2 (sehr schwach), 3 (schwach) und 4 (positiv) auch mit dem aufgetragenen PsaA-Antigen. Das Anti-Y. *pseudotuberculosis* -Serum aus Kaninchen reagierte sehr stark mit YopD und PsaA, zeigte aber keine Reaktivität mit dem Gesamtantigen MyfA oder dessen Teilfragmenten. Die in dem Assay auftretende Kreuzreaktivität der Anti-Y. *enterocolitica* -Seren mit PsaA wird möglicherweise durch semi-optimale Produktionsbedingungen verursacht (d.h. Aufreinigung über His-Taq, Pufferkonditionen oder zu hohe Antigenkonzentration). Zusätzlich sind auftretende Kreuzreaktivitäten zwischen MyfA und PsaA bzw. andere Yersinien-Oberflächenproteine (sog. RPs) in mit vorkultivierten Yersinien-immunisierten Kaninchenseren bekannt (Leiva et al., Heesemann et al.). Für folgende Experimente wurden die MyfA- und PsaA Antigene umkloniert (ohne His-Taq und Leader-Sequenz), aufgereinigt und die Assay-Konditionen optimiert (Beispiele 3-5).

### Beispiel 4

### Herstellung und Reinigung der rekombinanten MyfA- und PsaA-Antigene

Ausgehend von den vollständigen Leserahmen (Beispiel 1) der beiden Antigene wurden MyfA (p*myfA* MIK)- und PsaA (ppsaA-MIK)-Expressionsklone hergestellt. Nachfolgende Oligonukleotid-Primer wurden zur Darstellung der Sequenzen verwendet. Die Proteine wurden ohne Leader-Sequenz (MyfA AA 1-29 und PsaA AA 1-26; Beispiel 1) hergestellt, da die vorherigen Experimenten zeigten, dass das Leader-Peptid eine reduzierte Expression hervorruft.
***pmyfA-*MIK**
   Primer 5 (Seq ID No. 17):
      myfA-F-Ndel: CAC ATA TGG AAC CGA CTG TTA TTA ATA GTA AAG ACA TC
   Primer 6 (Seq ID No. 18):
      myfA-R-Baml: ATG GAT CCT TAC TCG ACA TAT TCC TCA ACA CG
***ppsaA*-MIK**
   Primer 7 (Seq ID No. 19):
      psaA-F-Ndel: GCC ATA TGT CTA CTG TCA TTA ACT CCA AGG ATG
   Primer 8 (Seq ID No. 20):
      psaA-R-Baml: CAG GAT CCT TAA AAT ACA TAC TCT TCA ACA CGC C

Die spezifische Amplifikation des *myfA*-Fragments erfolgte mittels PCR mit chromosomaler DNA aus *Y. enterocolitica* Serotyp O:3/4 (*Y. enterocolitica* subsp. *palearctica* Stamm Y-11; DMSZ 13030) als Template. Das *psaA*-Fragment wurde mit chromosomaler DNA aus *Y. pseudotuberculosis* Serotyp 1A amplifiziert.

Die resultierenden Amplifikate wurden mittels Restriktionsendonukleasen *Nde* I und *Bam* HI enzymatisch gespalten und in einen geeigneten Vektor, pET3c (New England Biolabs) ligiert. Nach der Transformation des Ligationsansatzes in den *E. coli*-Stamm UT 5600 (Elish et al. [1998] J. Gen. Microbiol., 134, S. 1355-1364) wurden die Klone auf das Vorhandensein der *myfA-* und *psaA* -Fragmente mittels Agarose-Gelelektrophorese von enzymatisch gespaltener Plasmid-DNA und mittels DNA-Sequenzierung (siehe unten) getestet. Des Weiteren wurde die Expression der Antigene durch Analyse der Expressionsprodukte MyfA-MIK und PsaA-MIK mittels SDS-Polyacrylamid-Gelelektrophorese mit anschließender Coomassie-Blau-Färbung bzw. anschließendem Transfer auf Nitrozellulose gefolgt von einer immunologischen Detektion identifiziert und charakterisiert.

Die Reinigung der rekombinanten Proteine MyfA-MIK und PsaA-MIK erfolgte mittels Anionenaustausch- und Kationenaustausch-Säulenchromatographie. Im ersten Schritt wurde ein Anionenaustausch (Q-Sepharose Fast Flow; GE Healthcare, München, Deutschland), im zweiten ein Kationenaustausch (S-Source 15; GE Healthcare) und im dritten ein Anionenaustausch (Q-Source 30; GE Healthcare) durchgeführt. Die einzelnen Reinigungsschritte bzw. das aufgereinigte Protein wurde mittels SDS-Polyacrylamid-Gelelektrophorese mit anschließender Coomassie-Blau-Färbung bzw. anschließendem Transfer auf Nitrozellulose gefolgt von einer immunologischen Detektion überprüft und hinsichtlich des Reinheitsgrads, möglicher Proteinspaltung und immunologischer Reaktivität charakterisiert.

### Beispiel 5

### DNA-Sequenzierung der pmyfA-MIK und ppsaA-MIK-Expressionsklone und daraus resultierende Proteinsequenzen (AA) MyfA-MIK und PsaA-MIK

Die START **(atg)**- und STOP **(uaa)**-Kodons sind schwarz markiert.

### DNA-Sequenzen

der ***pmyr*A-MIK** (396 bp) (Seq ID No. 21):
***ppsaA*-MIK** (402 bp) (Seq ID No. 22): **Aminosäuresequenzen (AA)**
**MyfA-MIK** (132-AA) (Seq ID No. 23)
**PsaA-MIK** (134-AA) (Seq ID No. 24)

### Beispiel 6

### Serologische Differenzierung der Yersiniosen verursacht durch Y. enterocolitica oder Y. pseudotuberculosis

Zwei humane Serumproben wurden mittels Widal-Reaktion als Anti-*Y. enterocolitica* Serotyp O:3 (LYE16)- bzw. als Anti-*Y. pseudotuberculosis* (LYE01) -IgG-positiv definiert.

Der Vergleich der serologischen IgG-Reaktivität von Yop-, MyfA-MIK- und PsaA-MIK-Antigenen (Beispiel 3) mittels Line-Assays zeigte, dass die *Y. pseudotuberculosis*-positive Serumprobe sehr stark mit YopD und PsaA reagierte. Die Anti-*Y. enterocolitica*-IgG-positive Probe reagierte hingegen mit den Antigenen YopM, YopH, V-AG, YopD, YopN, YopE und MyfA. Diese Serumprobe zeigte auch eine sehr schwache Reaktivität mit PsaA.

Somit ergibt sich ein Hinweis darauf, dass die Antigene MyfA-MIK und PsaA-MIK die serologische Differenzierung von *Y. enterocolitica-* und *Y. pseudotuberculosis*-Infektionen ermöglicht.

### Beispiel 7

### Vergleich der serologischen Prävalenz der Anti-Yop-, Anti-MyfA- und Anti-PsaA-Antikörper der IgG-, IgM- und IgA-Klasse in Serumproben von bayerischen Blutspendern bzw. in Serumproben von finnischen und deutschen Yersiniose-Patienten

Die IgG-, IgM- und IgA-Antwort gegen rekombinant hergestellte YopM-, YopH-, V-AG-, YopD, YopN-, YopE-, MyfA-MIK- und PsaA-MIK- (Beispiel 4 und 5) Antigene mit Seren aus drei verschiedenen Serensammlungen (d. h. bayerische Blutspender n=40; Bayern; Deutschland, Yersiniose-Patienten aus Finnland [KTL, n=18] und Yersiniose-Patienten aus Deutschland [YeD, n=23]) wurden mittels Line-Assays untersucht. Als Referenztest wurde der *recom*Line Yersinia der Fa. Mikrogen GmbH verwendet.

### Blutspenderseren

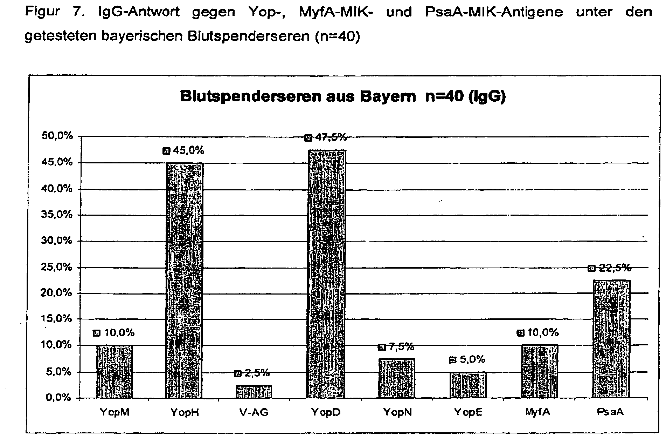

48-% (n=19) der getesteten Blutspenderseren aus Bayern (n=40) zeigten eine IgG-Reaktivität mit YopD (Fig. 7; Tabelle 1). Acht Serumproben wiesen eine IgG-Antwort auf, die nicht gegen YopD sondern ausschließlich gegen YopH (n=4), YopH und YopN (n=1), YopH und V-AG (n=1), YopM (n=1) und PsaA-MIK (n=1) gerichtet war. Drei der Proben reagierten mit den beiden neuen Yersinien-Antigene MyfA-MIK (n=4) und PsaA-MIK (n=9; Tab. 1).

Da die Seren von gesunden Spendern stammten, konnte in keinem der getesteten Seren IgM-Antikörper gegen YopD nachgewiesen werden (Tab. 1). Drei Yop-IgG-positive Seren (n=19) wiesen einen isolierten IgM-Titer gerichtet gegen YopH (n=2; 11 %; Serum Nr. 1087: hohe IgG-Titer gegen YopD und PsaA; Serum Nr. 1099: hohe IgG-Titer gegen YopD) oder PsaA (n=1; 5%, Serum Nr. 1083: hohe IgG-Titer gegen YopD, MyfA-MIK und PsaA-MIK) auf. Keine der getesteten Proben reagierte mit MyfA-MIK (Tab. 1).

Sieben YopD-IgG-positive Serumproben aus Blutspendern (n=19) zeigten einen IgA-Titer gegen YopD (Tab. 1). Drei der getesteten Seren reagierten mit PsaA-MIK. Interessanterweise zeigte die Yop-IgG-negative Serumprobe Nr. 1080 eine sehr schwache isolierte Reaktivität mit PsaA. Die YopD- und PsaA-MIK IgG-hoch positiven Serumproben Nr. 1096 und Nr. 1110 wiesen auch eine starke (Serum Nr. 1096) bzw. schwache (Serum Nr. 1110) IgA-Antwort gegen PsaA-MIK (Tab. 1) auf. Keiner der getesteten Proben reagierte mit MyfA-MIK (Tab. 1). Aufgrund der parallel auftretenden Anti-IgA-Reaktivität gegen die YopD-, Yop-N- und PsaA- Antigene in den Proben Nr. 1110 ist es zu erwarten, dass es hier um einen diagnostisch relevanten serologischen Befund handelt, wie z.B. kürzliche *Y.pseudotuberculosis*-Infektion bzw. auftretende reaktive Arthritis.

### Yersiniose-Patientenseren aus Finnland

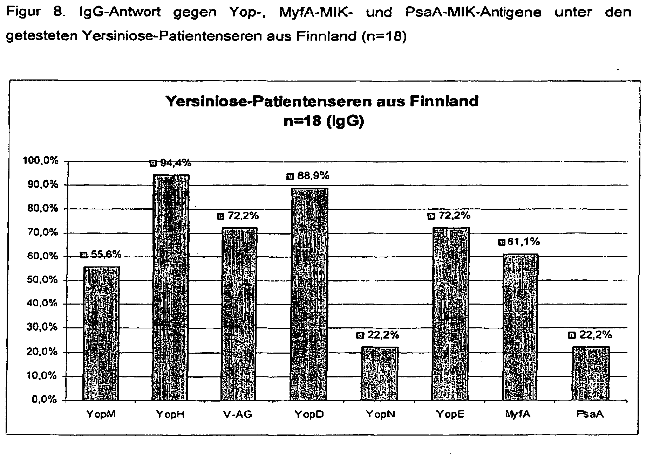

17 (90-%) der getesteten Seren aus Patienten mit Verdacht auf Yersinien-induzierte reaktive Arthritis aus Finnland (n=18) zeigten eine IgG-Antwort gegen YopD (Fig. 8; Tab. 2). Elf der Proben reagierten mit MyfA-MIK (65 %) und vier mit PsaA-MIK (24%) (Fig. 8; Tab. 2).
15 (88%) der getesteten Seren hatten IgM-Antikörper gegen YopD (Tab. 2). Ein Serum (Nr. 52) zeigte auch eine starke IgM-Antwort gegen PsaA-MIK (Tab. 2).
17 Serumproben aus Yersiniose-Patienten (n=18) wiesen einen IgA-Titer gegen YopD (Tab. 2) auf. Drei der getesteten Seren reagierten auch mit PsaA-MIK (Nr. 23, 24 und 40; Tab. 2).

### Yersiniose-Patientenseren aus Deutschland

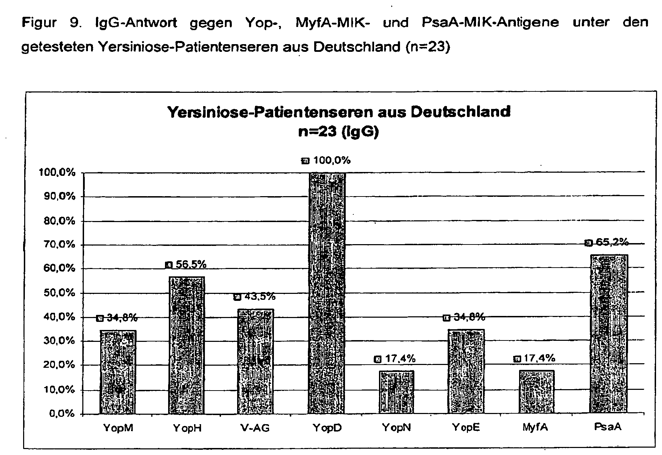

Alle (n=23) getesteten Yersiniose-Patientenseren aus Deutschland zeigten eine IgG-Reaktivität mit YopD (Fig. 9; Tab. 3). 15 Serumproben (65%) wiesen zusätzlich eine IgG-Antwort gegen PsaA-MIK auf. Vier (17 %) der Proben reagierten mit dem Antigen MyfA-MIK (Fig. 9; Tab. 3). Interessanterweise reagierten zwei der Serumproben (Serum 1038 und 1025) mit den beiden Antigenen MyfA-MIK und PsaA-MIK. Jedoch war die IgG-Antwort gegen MyfA-MIK deutlich schwächer im Vergleich zu PsaA-MIK (Tab. 3).

Nur in zwei (9 %) der getesteten Seren (n=23) wurde eine niedrige IgM-Antwort gegen YopD und in drei Seren gegen YopE nachgewiesen (Tab. 3). Ein Serum, Nr. 986, reagierte sehr stark mit PsaA-MIK (Tab. 3).

Alle getesteten Yersiniose-Patientenseren wiesen eine IgA-Antwort gegen YopD (Tab. 3) auf. Eine der Proben reagierte auch mit MyfA-MIK (Serum Nr. 976) und eine andere mit PsaA-MIK (Serum Nr. 1025). Diese Seren zeigten auch eine starke IgG-Antwort gegen MyfA-MIK bzw. PsaA-MIK (Tab. 3).

Interessanterweise unterscheiden sich die zwei getesteten Yersiniose-Patientenprobensammlungen aus Finnland und Deutschland in der Anti-MyfA- und Anti-PsaA-IgG-Prävalenz. Aufgrund niedriger Isolierungsrate bzw. technisch schwerer Kultivierung und Isolierung der *Y.pseudotuberculosis*-Stämme sind momentan keine zuverlässigen epidemiologischen Daten vorhanden.

**Tabelle 1. Die IgG-, IgM- und IgA-Antwort gegen rekombinant hergestellte YopM-, YopH-, V-AG-, YopD, YopN-, YopE-, MyfA-MIK- und PsaA-MIK-Antigene unter den getesteten Blutspenderseren aus Bayern (n=40). Die Reaktivität wurde semi-quantitativ dargestellt mit folgendem Auswertungsschema: 1 = sehr schwache Reaktivität, 2 = schwache Reaktivität, 3 und 4 = starke Reaktivität.**

| **Serum** | **YopM** | **YopH** | **V-AG** | **YopD** | **YopN** | **YopE** | **MyfA** | **PsaA** |
|---|---|---|---|---|---|---|---|---|
| **IgG-Antwort** | | | | | | | | |
| **1075** | 1 | 4 | | 4 | | 2 | 4 | 4 |
| **1076** | | 1 | | | 1 | | | |
| **1077** | | 2 | | 4 | | 4 | 2 | |
| **1078** | | | | | | | | |
| **1079** | | | | | | | | |
| **1080** | | | | | | | | |
| **1081** | | | | | | | | |
| **1082** | | 1 | | 3 | | | | |
| **1083** | | | | 4 | 1 | | 4 | 2 |
| **1084** | | | | | | | | |
| **1085** | | 3 | | 1 | | | | |
| **1086** | | 3 | | 3 | | | | |
| **1087** | | | | 4 | | | | 4 |
| **1088** | | | | | | | | |
| **1089** | | | | | | | | |
| **1090** | | 2 | | | | | | |
| **1091** | | 2 | | | | | | |
| **1092** | | | | | | | | |
| **1093** | 2 | 1 | | 4 | | | | |
| **1094** | | | | | | | | 2 |
| **1095** | | | | | | | | |
| **1096** | | | | 3 | | | 3 | 4 |
| **1097** | | 1 | | 3 | | | | |
| **1098** | | 1 | | 1 | | | | |
| **1099** | | | | 3 | | | | |
| **1100** | | | | | | | | |
| **1101** | 1 | 3 | | 2 | | | | |
| **1102** | | 3 | | 2 | | | | 3 |
| **1103** | | 3 | | | | | | |
| **1104** | | 1 | | | | | | |
| **1105** | | | | | | | | |
| **1106** | | | | | | | | |
| **1107** | 1 | | | | | | | |
| **1108** | | | | 1 | | | | 3 |
| **1109** | | 2 | | 2 | 2 | | | |
| **1110** | | | | 2 | | | | 3 |
| **1111** | | | | | | | | |
| **1112** | | 2 | | 4 | | | | 2 |
| **1113** | | 2 | 2 | | | | | |
| **1114** | | | | 4 | | | | |

| **IgM-Antwort** | | | | | | | | |
|---|---|---|---|---|---|---|---|---|
| **1075** | | | | | | | | |
| **1076** | | | | | | | | |
| **1077** | | | | | | | | |
| **1078** | | | | | | | | |
| **1079** | | | | | | | | |
| **1080** | | | | | | | | |
| **1081** | | | | | | | | |
| **1082** | | | | | | | | |
| **1083** | | | | | | | | 2 |
| **1084** | | | | | | | | |
| **1085** | | | | | | | | |
| **1086** | | | | | | | | |
| **1087** | | 1 | | | | | | |
| **1088** | | | | | | | | |
| **1089** | | | | | | | | |
| **1090** | | | | | | | | |
| **1091** | | | | | | | | |
| **1092** | | | | | | | | |
| **1093** | | | | | | | | |
| **1094** | | | | | | | | |
| **1095** | | | | | | | | |
| **1096** | | | | | | | | |
| **1097** | | | | | | | | |
| **1098** | | | | | | | | |
| **1099** | | 4 | | | | | | |
| **1100** | | | | | | | | |
| **1101** | | | | | | | | |
| **1102** | | | | | | | | |
| **1103** | | | | | | | | |
| **1104** | | | | | | | | |
| **1105** | | | | | | | | |
| **1106** | | | | | | | | |
| **1107** | | | | | | | | |
| **1108** | | | | | | | | |
| **1109** | | | | | | | | |
| **1110** | | | | | | | | |
| **1111** | | | | | | | | |
| **1112** | | | | | | | | |
| **1113** | | | | | | | | |
| **1114** | | | | | | | | |

| **IgA-Antwort** | | | | | | | | |
|---|---|---|---|---|---|---|---|---|
| **1075** | | | | | | | | |
| **1076** | | | | | | | | |
| **1077** | | | | 1 | | | | |
| **1078** | | | | | | | | |
| **1079** | | | | | | | | |
| **1080** | | | | | | | | 1 |
| **1081** | | | | | | | | |
| **1082** | | | | 1 | | | | |
| **1083** | | | | | | | | |
| **1084** | | | | | | | | |
| **1085** | | | | | | | | |
| **1086** | | | | | | | | |
| **1087** | | | | 4 | | | | |
| **1088** | | | | | | | | |
| **1089** | | | | | | | | |
| **1090** | | | | | | | | |
| **1091** | | | | | | | | |
| **1092** | | | | | | | | |
| **1093** | | | | 1 | | | | |
| **1094** | | | | | | | | |
| **1095** | | | | | | | | |
| **1096** | | | | | | | | 3 |
| **1097** | | | | 3 | | | | |
| **1098** | | | | | | | | |
| **1099** | | | | | | | | |
| **1100** | | | | | | | | |
| **1101** | | | | | | | | |
| **1102** | | | | | | | | |
| **1103** | | | | | | | | |
| **1104** | | | | | | | | |
| **1105** | | | | | | | | |
| **1106** | | | | | | | | |
| **1107** | | | | | | | | |
| **1108** | | | | | | | | |
| **1109** | | | | | | | | |
| **1110** | | | | 3 | 2 | | | 1 |
| **1111** | | | | | | | | |
| **1112** | | | | | | | | |
| **1113** | | | | | | | | |
| **1114** | | | | 1 | | | | |

**Tabelle 2. Die IgG-, IgM- und IgA-Antwort gegen rekombinant hergestellte YopM-, YopH-, V-AG-, YopD, YopN-, YopE-, MyfA-MIK- und PsaA-MIK -Antigene unter den getesteten Yersiniose-Patientenseren aus Finnland (n=18). Die Reaktivität wurde semi-quantitativ dargestellt mit folgendem Auswertungsschema: 1 = sehr schwache Reaktivität, 2 = schwache Reaktivität, 3 und 4 = starke Reaktivität.**

| | **YopM** | **YopH** | **V-AG** | **YopD** | **YopN** | **YopE** | **MyfA** | **PsaA** |
|---|---|---|---|---|---|---|---|---|
| **IgG-Antwort** | | | | | | | | |
| **1** | 1 | 3 | 3 | 4 | 2 | 3 | 1 | |
| **5** | 2 | 4 | 1 | 4 | | 1 | 3 | |
| **6** | | 1 | 1 | 4 | | | | |
| **11** | | 3 | 1 | 3 | | 2 | 1 | |
| **16** | | 2 | 2 | 3 | | | | |
| **19** | | 3 | | 4 | | 2 | | |
| **23** | | 2 | | 3 | | | | 3 |
| **24** | 2 | 4 | 3 | 4 | | 3 | 1 | 4 |
| **34** | | 3 | | 3 | | 1 | 2 | |
| **35** | 2 | 3 | 3 | 4 | | 1 | | |
| **36** | 2 | 2 | 2 | 4 | | 1 | 1 | |
| **40** | 3 | 3 | 3 | 4 | 1 | 2 | 3 | 1 |
| **44** | | 3 | 1 | 3 | | 1 | | |
| **47** | | | | | | | | |
| **50** | 2 | 3 | | 4 | | 3 | 1 | 2 |
| **51** | 3 | 4 | 3 | 4 | 3 | 3 | 1 | |
| **52** | 1 | 4 | 2 | 4 | 2 | 3 | 1 | |
| **53** | 3 | 4 | 2 | 4 | | | 1 | |

| **IgM-Antwort** | | | | | | | | |
|---|---|---|---|---|---|---|---|---|
| **1** | | 1 | | 4 | | | | |
| **5** | | | | 3 | | | | |
| **6** | | | | 2 | | | | |
| **11** | | | | 1 | | | | |
| **16** | | | | 1 | | | | |
| **19** | | | | 3 | | | | |
| **23** | | | | | | | | |
| **24** | | | | 3 | | | | |
| **34** | | | | | | | | |
| **35** | | | | 4 | | | | |
| **36** | | | | 3 | | | | |
| **40** | | | | 1 | | | | |
| **44** | | | | 2 | | | | |
| **47** | | | | | | | | |
| **50** | | | | 1 | | | | |
| **51** | | | | 3 | | | | |
| **52** | | | | 2 | | | | 3 |
| **53** | | | | 4 | | | | |

| **IgA-Antwort** | | | | | | | | |
|---|---|---|---|---|---|---|---|---|
| **1** | | | | 2 | | | | |
| **5** | | | | 2 | | | | |
| **6** | | | | 4 | | | | |
| **11** | | | | 1 | | | | |
| **16** | | | | 2 | | | | |
| **19** | | | | 2 | | | | |
| **23** | | | | 2 | | | | 4 |
| **24** | | 1 | | 4 | | | | 3 |
| **34** | | | | 2 | | | | |
| **35** | | | | 3 | | | | |
| **36** | | | | 3 | | | | |
| **40** | 1 | 2 | 1 | 4 | 2 | 2 | | 1 |
| **44** | | 1 | | 4 | | | | |
| **47** | | | | | | | | |
| **50** | | | | 3 | | | | |
| **51** | | 1 | | 2 | | | | |
| **52** | | | | 2 | 1 | | | |
| **53** | | 3 | | 4 | | | | |

**Tabelle 3. Die IgG-, IgM- und IgA-Antwort gegen rekombinant hergestellte YopM-, YopH-, V-AG-, YopD, YopN-, YopE-, MyfA-MIK- und PsaA-MIK -Antigene unter den getesteten Yersiniose-Patientenseren aus Deutschland (n=23). Die Reaktivität wurde semi-quantitativ dargestellt mit folgendem Auswertungsschema: 1 = sehr schwache Reaktivität, 2 = schwache Reaktivität, 3 und 4 = starke Reaktivität.**

| | **YopM** | **YopH** | **V-AG** | **YopD** | **YopN** | **YopE** | **MyfA** | **PsaA** |
|---|---|---|---|---|---|---|---|---|
| **IgG-Antwort** | | | | | | | | |
| **262** | | | | 2 | | | | 2 |
| **327** | | 4 | 2 | 4 | 3 | 2 | | 2 |
| **1534** | 1 | 3 | 3 | 4 | 1 | 4 | 3 | |
| **976** | | 4 | | 4 | | 4 | 2 | |
| **1038** | 2 | 3 | 2 | 3 | | | 1 | 2 |
| **158** | 2 | 3 | 1 | 4 | | 2 | | 3 |
| **58** | 2 | 3 | | 4 | | | | |
| **321** | | | | 4 | | | | 2 |
| **326** | | 4 | 2 | 4 | 2 | 2 | | 2 |
| **333** | | | 1 | 4 | | | | 2 |
| **1025** | | 3 | | 4 | | | 1 | 4 |
| **1547** | | | | 1 | | | | |
| **111** | | | | 4 | | | | 2 |
| **1** | 3 | 4 | 3 | 4 | | 4 | | 3 |
| **252** | 1 | 4 | 2 | 4 | 2 | 4 | | |
| **262** | | | | 3 | | | | 3 |
| **113** | | 4 | 2 | 4 | | 3 | | |
| **986** | | 4 | | 4 | | | | |
| **320** | 2 | | | 2 | | | | |
| **1748** | 1 | | 1 | 4 | | | | 3 |
| **1176** | | 3 | | 3 | | | | 3 |
| **1801** | | | | 4 | | | | 3 |
| **1810** | | | | 4 | | | | 3 |

| **IgM-Antwort** | | | | | | | | |
|---|---|---|---|---|---|---|---|---|
| | | | | | | | | |
| **262** | | | | | | | | |
| **327** | | | | | | | | |
| **1534** | | | | | | | | |
| **976** | | | | | | | | |
| **1038** | | | | | | 1 | | |
| **158** | | | | | | 1 | | |
| **58** | | | | | | 1 | | |
| **321** | | | | 1 | | | | |
| **326** | | | | | | | | |
| **333** | | | | 1 | | | | |
| **1025** | | | | | | | | |
| **1547** | | | | | | | | |
| **111** | | | | | | | | |
| **1** | | | | | | | | |
| **252** | | | | | | | | |
| **262** | | | | | | | | |
| **113** | | | | | | | | |
| **986** | | | | | | | | 4 |
| **320** | | | | | | | | |
| **1748** | | | | | | | | |
| **1176** | | | | | | | | |
| **1801** | | | | | | | | |
| **1810** | | | | | | | | |

| **IgA-Antwort** | | | | | | | | |
|---|---|---|---|---|---|---|---|---|
| **262** | | | | 2 | | | | |
| **327** | | | | 2 | | | | |
| **1534** | | | | 2 | | | | |
| **976** | | | | 2 | | | 1 | |
| **1038** | | | | 2 | | | | |
| **158** | | | | 3 | | | | |
| **58** | | | | 2 | | | | |
| **321** | | | | 3 | | | | |
| **326** | | | | 2 | | | | |
| **333** | | | | 3 | | | | |
| **1025** | | | | 2 | | | | 3 |
| **1547** | | | | 1 | | | | |
| **111** | | | | 3 | | | | |
| **1** | 3 | 3 | | 3 | | | | |
| **252** | | | | 2 | | | | |
| **262** | | | | 3 | | | | |
| **113** | | | | 3 | | | | |
| **986** | | | | 2 | | | | |
| **320** | 2 | | | 1 | | | | |
| **1748** | | | | 3 | | 2 | | |
| **1176** | | | | 4 | | | | |
| **1801** | | | | 4 | | | | |
| **1810** | | | | 4 | | | | |

### Beispiel 8

### Diagnostische Relevanz der PsaA-MIK- und MyfA-MIK -Antigene bei der Diagnose von akuten Y. pseudotuberculosis -Infektionen

Die verwendete Abkürzung "MIK" besagt, dass die Antigene von der Anmelderin rekombinant hergestellt werden.

Die PsaA-MIK- und MyfA-MIK -Antigene wurde eingesetzt in einen bead -basiertem Testsystem (Luminex) zum Nachweis einer IgG-, IgM- und IgA -Reaktivität gegen das PsaA-M IK- bzw. das MyfA-MIK -Antigen. Eine Sammlung von Serumproben von Patienten, die an einer akuten, durch Yersinia pseudotuberculosis verursachten Yersiniose erkrankt waren (K. Jalava, P. Nuorti: "Porkkanaraasteesta laaja Yersinia pseudotuberculosis -epidemia, Kansanterveyslehti, 2003) wurde eingesetzt. Als negative Vergleichsgruppe wurden 8 *Y. enterocolitica* IgG -positive (bestimmt mit sog. Widal-Hemagglutinationstest und ein Blot -Assay) und 19 *Y. enterocolitica* -negative Routineproben eingesetzt. Die Ergebnisse sind in Tabelle 4 zusammengefaßt.

96.9% (63 Proben aus 65 getesteten Proben) der getesteten *Y*. *pseudotuberculosis*-Patientenproben zeigten hohe IgG -Reaktivität gegen das PsaA-MIK Antigen. Nur drei der getesteten Proben reagierten parallel gegen beide Antigene (MyfA-MIK- und PsaA-MIK). 44.6% der Proben wiesen eine Anti -PsaA-MIK -IgM - und 58.5% der Proben eine Anti -PsaA-MIK -IgA -Reaktivität auf. Keine der getesteten Proben reagierte mit den MyfA-MIK -Antigen, wenn Antikörper der IgM- und IgA -Klassen untersucht wurden.

75.0% der *Y. enterocolitica* WIDAL- und Blot -positiven Proben waren IgG -positiv für das MyfA-MIK -Antigen. Keine dieser Proben reagierte mit dem PsaA-MIK -Antigen. Keine dieser Proben zeigte eine IgM- oder IgA -Reaktivität gegen die PsaA-MIK- oder MyfA-MIK -Antigene.

Keine der Yersinia -IgG -negativen Proben (n=19) reagierte mit dem PsaA-MIK -Antigen. Drei der Proben zeigten isolierte Anti -MyfA-MIK -IgG -Reaktivität und zwei der Proben isolierte Anti -PsaA-MIK -IgM -Reaktivität.

**Tabelle 4. Untersuchung der Anti- PsaA-MIK und -MyfA-MIK IgG-, IgM- und IgA-Reaktivität bei Y. pseudotuberculosis -verursachten Yersiniose -Patienten und bei Y. enterocolitica -IgG -positiven bzw. Yersinia -IgG -negativen Proben.**

| Probensammlung | IgG | | IgM | | IgA | |
|---|---|---|---|---|---|---|
| | PsaA | MyfA | PsaA | MyfA | PsaA | MyfA |
| *Y. pseudotuberculosis-*Patientenproben n=65 | 63 (96.9%) | 3 (4.6%) | 29 (44.6%) | 0 | 38 (58.5%) | 0 |
| *Y. enterocolitica* IgG - positiv n=8 | 0 | 6 (75.0%) | 0 | 0 | 0 | 0 |
| Yersinia IgG -negativ n = 19 | 0 | 3 (15.8%) | 2 (10.5%) | 0 | 0 | 0 |

Das vorliegende Beispiel belegt, dass überraschenderweise die Antigene PsaA bzw. MyfA die Unterscheidung von Infektionen mit *Y.pseudotuberculosis* von Infektionen mit *Y.enterocolitica* ermöglichen, obwohl verhältnismäßig hohe Homologien zwischen den beiden Antigenen vorhanden sind, die auch zu Kreuzreaktivitäten führen können. Kreuzreaktivitäten zu MyfA- bzw. PsaA-ähnlichen Antigenen, die in anderen Enterobakterien vorkommen, können durch Verwendung der anderen hier beschriebenen Antigene, insbesondere Yop D, ausgeschlossen werden.

Aufgrund dieser Ergebnisse ist anzunehmen, dass die hier beschriebene Vorrichtung auch zum serologischen Nachweis einer Infektion verursacht durch *Y.pestis* eingesetzt werden könnte. Da aber eine *Y.pestis*-Infektion - im Gegensatz zu einer *Y.pseudotuberculosis*-Infektion typischerweise mit einer schwarzen, schnell voranschreitenden, anders verlaufenden Symptomatik assoziiert ist (sog. Lungenpest, Beulenpest), erlaubt eine Serodiagnose unterstützt mit klinischer Symptomatik eine zuverlässige Unterscheidung der beiden Infektionstypen.

## Patentansprüche

1. Vorrichtung zur serologischen Unterscheidung einer Infektion mit *Yersinia enterocolitica* von einer Infektion mit *Yersinia pseudotuberculosis*, **dadurch gekennzeichnet, dass** diese Vorrichtung
wenigstens ein Antigen, ausgewählt aus der Gruppe von Antigenen bestehend aus folgender Gruppe: YopD, YopH, YopM, YopE, V-AG und YopN oder ein Fragment eines dieser Antigene mit mindestens 20 aufeinanderfolgenden Aminosäuren, wobei jedes Peptidfragment wenigstens ein diagnostisch relevantes Epitop aufweist, und weiterhin sowohl das Protein MyfA, wie auch das Protein PsaA oder Fragmente dieser beiden Proteine mit mindestens 20 aufeinanderfolgenden Aminosäuren aufweist, wobei jedes Proteinfragment wenigstens ein diagnostisch relevantes Epitop aufweist und die einzelnen Antigene räumlich getrennt voneinander angeordnet sind, wobei es sich um einen diagnostischen Kit, nämlich um einen ELISA-Test, bei dem die einzelnen Antigene in verschiedene Kavitäten der Mikrotiterplatte eingebracht sind, um einen Line-Test, um Immunoblots, bei dem die diagnostisch relevanten Proteine der Größe nach aufgetrennt sind, Bead-Based-Assays oder Mikroarrays handelt.

2. Vorrichtung nach Anspruch 1, **dadurch gekennzeichnet, dass** die Fragmente der als Antigen eingesetzten Proteine wenigstens 30 Aminosäuren des jeweiligen Antigens aufweisen.

3. Vorrichtung nach Anspruch 1, **dadurch gekennzeichnet, dass** diese das Antigen YopD, YopH, YopM zusammen mit MyfA und PsaA beinhaltet.

4. Vorrichtung nach Anspruch 1, **dadurch gekennzeichnet, dass** die Vorrichtung das Antigen YopD, YopH, YopM, YopE zusammen mit dem Antigen PsaA und MyfA beinhaltet.

5. Vorrichtung nach Anspruch 1, **dadurch gekennzeichnet, dass** diese die Antigene YopD, YopH, YopM, YopE, V-AG, YopN sowie MyfA und PsaA beinhaltet

6. Verwendung des antigenen Proteins MyfA und/oder PsaA oder eines Fragmentes eines dieser Proteine mit wenigstens 20 aufeinanderfolgenden Aminosäuren, das ein diagnostisch relevantes Epitop aufweist, zur serologischen Unterscheidung einer durch *Yersinia enterocolitica* verursachten Infektion von einer durch *Yersinia pseudotuberculosis* verursachten Infektion, wobei die diagnostische Zuordnung zu den Yersinien durch immunologische Reaktion mit wenigstens einem Antigen ausgewählt aus der Gruppe Yop D, Yop H, Yop M, Yop E, V-AG und Yop N oder einem Fragment eines dieser Proteine mit mindestens 20 aufeinanderfolgenden Aminosäuren erfolgt und wobei jedes Antigen mindestens ein diagnostisch relevantes Epitop aufweist, **dadurch gekennzeichnet, dass** die Antigene in räumlich voneinander getrennter Form in einer Vorrichtung gemäß Ansprüchen 1-5 eingesetzt werden.

## Claims

1. Device for serologically differentiating an infection with *Yersinia enterocolitica* from an infection with *Yersinia pseudotuberculosis*, **characterised in that** said device has at least one antigen, selected from the group of antigens consisting of the following group: YopD, YopH, YopM, YopE, V-AG and YopN or a fragment of one of these antigens with at least 20 consecutive amino acids, wherein each peptide fragment has at least one diagnostically relevant epitope, and furthermore comprises the protein MyfA as well as the protein PsaA or fragments of these two proteins with at least 20 consecutive amino acids, wherein each protein fragment has at least one diagnostically relevant epitope and the individual antigens are arranged spatially separate from one another, this device being a diagnostic kit, namely an ELISA assay, wherein the individual antigens are inserted in different cavities of the microtiter plate, a Line assay, immunoblots, wherein the diagnostically relevant proteins are separated according to their size, bead-based assays or microarrays.

2. Device according to Claim 1, **characterised in that** the fragments of the proteins used as antigen have at least 30 amino acids of the respective antigen.

3. Device according to Claim 1, **characterised in that** it comprises the antigen YopD, YopH, YopM in combination with MyfA and PsaA.

4. Device according to Claim 1, **characterised in that** the device comprises the antigen YopD, YopH, YopM, YopE in combination with the antigen PsaA and MyfA.

5. Device according to Claim 1, **characterised in that** it comprises the antigens YopD, YopH, YopM, YopE, V-AG, YopN as well as MyfA and PsaA.

6. Use of the antigenic protein MyfA and/or PsaA or a fragment of one of these proteins with at least 20 consecutive amino acids having a diagnostically relevant epitope for serologically differentiating an infection caused by *Yersinia enterocolitica* from an infection caused by *Yersinia pseudotuberculosis,* wherein the diagnostic attribution to the Yersinia is effected by immunological reaction with at least one antigen selected from the group Yop D, Yop H, Yop M, Yop E, V-AG and Yop N or a fragment of one of these proteins with at least 20 consecutive amino acids and wherein each antigen has at least one diagnostically relevant epitope, **characterised in that** the antigens are used in a device according to claims 1-5 in a form spatially separate from one another.

## Revendications

1. Dispositif pour la différenciation sérologique d'une infection à *Yersinia enterocolitica* d'une infection à *Yersinia pseudotuberculosis*, **caractérisé en ce que** ce dispositif
contient au moins un antigène sélectionné dans le groupe des antigènes constitué par le groupe suivant: YopD, YopH, YopM, YopE, V-AG et YopN ou un fragment d'un de ces antigènes comprenant au moins 20 acides aminés successifs, chaque fragment peptidique présentant au moins un épitope pertinent pour le diagnostic, et
présente en outre aussi bien la protéine MyfA que la protéine PsaA ou des fragments de ces deux protéines comprenant au moins 20 acides aminés successifs, chaque fragment protéique présentant au moins un épitope pertinent pour le diagnostic et les différents antigènes étant disposés spatialement séparés les uns des autres, ce dispositif étant un kit de diagnostic, à savoir un test ELISA, dans lequel les antigènes individuels sont insérés dans des cavités différentes de la plaque microtitre, un test à lignes, des immunotransferts, dans lesquels les protéines pertinentes pour le diagnostic sont séparées selon leur taille, des essais à base de billes ou de microréseau.

2. Dispositif selon la revendication 1, **caractérisé en ce que** les fragments des protéines utilisées comme antigène présentent au moins 30 acides aminés de l'antigène concerné.

3. Dispositif selon la revendication 1, **caractérisé en ce que** celui-ci contient l'antigène YopD, YopH, YopM conjointement avec MyfA et PsaA.

4. Dispositif selon la revendication 1, **caractérisé en ce que** le dispositif contient l'antigène YopD, YopH, YopM, YopE conjointement avec l'antigène PsaA et MyfA.

5. Dispositif selon la revendication 1, **caractérisé en ce que** celui-ci contient les antigènes YopD, YopH, YopM, YopE, V-AG et YopN ainsi que MyfA et PsaA.

6. Utilisation de la protéine antigène MyfA et/ou PsaA ou d'un fragment d'une de ces protéines comprenant au moins 20 acides aminés successifs, qui présente un épitope pertinent pour le diagnostic, pour la différenciation sérologique d'une infection due à *Yersinia enterocolitica* d'une infection due à *Yersinia pseudotuberculosis*, la classification diagnostique dans les *Yersinia* ayant lieu par réaction immunologique avec au moins un antigène sélectionné dans le groupe Yop D, Yop H, Yop M, Yop E, V-AG et Yop N ou un fragment d'une de ces protéines comprenant au moins 20 acides aminés successifs et chaque antigène présentant au moins un épitope pertinent pour le diagnostic, **caractérisée en ce que** les antigènes sont utilisés sous forme spatialement séparés les uns des autres dans un dispositif selon les revendications 1 à 5.
